# EUROPÄISCHE PATENTSCHRIFT

(11) **EP 1 308 122 B1**
(45) Veröffentlichungstag und Bekanntmachung des Hinweises auf die Patenterteilung: **02.08.2006**
(21) Anmeldenummer: 02024140.2
(22) Anmeldetag: 30.10.2002
(51) Int. Cl.: A61B 1/04

(54) **Vorrichtung zur bildgebenden Diagnose von Gewebe**
Device for the picture-providing diagnosis of tissue
Dispositif d'imagerie pour le diagnostic tissulaire

(30) Priorität: 02.11.2001 DE 10153900
(43) Veröffentlichungstag der Anmeldung: 07.05.2003
(73) Patentinhaber: Richard Wolf GmbH, 75438 Knittlingen (DE)
(72) Erfinder: Weber, Bernd Claus, Dr. Dipl.-Ing., 76133 Karlsruhe (DE); Goll, Thomas, 75438 Knittlingen (DE); Pereira-Delgado, Nicolas, 75433 Maulbronn (DE); Eidner, Philipp, 75015 Bretten-Büchig (DE); Müller, Stefan, Dipl.-Ing. (FH), 75015 Bretten-Diedelsheim (DE); Schmidt, Olaf, Dipl.-Ing., 71665 Vaihingen/Enz (DE); Wagniéres, Georges, Dr., 1095 Lutry (CH); Glanzmann, Thomas, Dr., 4102 Binningen (CH); van den Bergh, Hubert, Prof. Dr., 1025 St. Sulpice (CH); Gabrecht, Tanja, 58739 Wickede (DE)
(74) Vertreter: Hemmer, Arnd

(56) Entgegenhaltungen:
- WO-A-00/42910
- WO-A-01/45557
- US-A- 5 590 660
- US-A- 5 772 580
- US-A- 6 110 106
- US-A1- 2002 147 383

## Beschreibung

Die Erfindung betrifft eine Vorrichtung zur bildgebenden Diagnose von Gewebe unter wahlweiser Anwendung von zwei Diagnosemethoden, nämlich einem Arbeitsmodus zur bildgebenden Weißlichtdiagnose und einem Arbeitsmodus zur bildgebenden Autofluoreszenzdiagnose, mit einer Lichtquelle, deren Licht über ein Endoskop zum Gewebe geleitet wird, und mit einer Bildübertragungseinheit und einer Bildaufnahmeeinheit, welche mit einer Bildverarbeitungseinheit in Verbindung steht, über welche ein Monitor mit einem Bildsignal versorgt wird.

Die Therapie von prä- bzw. frühmalignen Läsionen verspricht die besten Heilungschancen, während die Erfolgsaussichten bei einem kurativen Behandlungsversuch ab dem Stadium des invasiven Karzinoms rapide abnehmen. Daher kommt der Diagnose von prä- bzw. frühmalignem Gewebe eine hohe Bedeutung zu.

Der Prozess der Karzinogenese von den ersten Zellatypien bis zum Stadium des invasiven Karzinoms erstreckt sich über einen Zeitraum von mehreren Jahren. Diese prä- und frühmaligne Phase bietet daher ein großes diagnostisches Zeitfenster, in welchem eine erfolgreiche Behandlung des Patienten und eine Tumorprävention gegeben sind. Leider konnte dieser Zeitrahmen für therapeutische Zwecke bisher nur unzureichend genutzt werden, was sich in der seit Jahrzehnten nahezu unverändert geringen Fünf-Jahres-Überlebensrate bei Patienten mit Bronchialkarzinom widerspiegelt. Diese Überlebensrate liegt nach wie vor nur zwischen 10 % und 15%.

Der Grund für diese bisher unzureichenden Nutzungsmöglichkeiten im gegebenen langen Zeitrahmen liegt darin, dass die Sensitivität der konventionellen Diagnoseformen - dazu zählt auch die Weißlichtendoskopie - für prä- und frühmaligne Läsionen sehr gering ist. Dementsprechend kann die Detektion bzw. Lokalisierung und daran anschließend eine Therapie der Läsionen nicht oder nur in wenigen Fällen erfolgen.

Bei der Untersuchung eines Patienten im Bronchialbereich ist es für den untersuchenden Arzt wünschenswert, zunächst eine gewöhnliche Weißlichtdiagnose durchzuführen, um sich dabei unter Weißlicht einen Über blick zu verschaffen und eine Vorabdiagnose zu erstellen. Hierbei wird nach entzündetem und malignem Gewebe, wie zum Beispiel exophytischen Tumoren, aber auch nach frühmalignem Gewebe gesucht, soweit dieses bereits unter Weißlicht sichtbar ist.

Bei der konventionellen Weißlichtdiagnose ist jedoch häufig prä- und frühmalignes Gewebe nicht von benignem Gewebe zu unterscheiden, weshalb es nicht aufgefunden werden kann. Die Weißlichtdiagnose hat hierfür eine unzureichende Sensitivität. Dementsprechend kann die Notwendigkeit einer lebensrettenden oder zumindest deutlich lebensverlängernden Therapie nicht erkannt werden. Hier stellt die bildgebende Autofluoreszenzdiagnose einen entscheidenden Vorteil dar: Sie verbessert die Sichtbarmachung bzw. die Möglichkeit der Differenzierung von prä- und frühmalignem Gewebe gegenüber gesundem, entzündetem oder lediglich metaplastisch verändertem Gewebe wesentlich.

Dabei ist hinsichtlich der bildgebenden Autofluoreszenzdiagnose bei der Diagnose von prä- bzw. frühmalignen Läsionen zu deren Unterscheidung von gesundem Gewebe folgendes festzustellen: Gesundes Gewebe unterscheidet sich von krankem Gewebe zum einen in der Fluoreszenz intensität integral über den gesamten Emissionsbereich betrachtet, d. h. in der Helligkeit, und zum anderen in der Fluoreszenzfarbe (beim Betrachter hervorgerufener integraler Farbeindruck, verursacht durch eine wechsefnde Gewichtung der spektralen Anteile im vom Gewebe abgegebenen Fluoreszenzlicht).

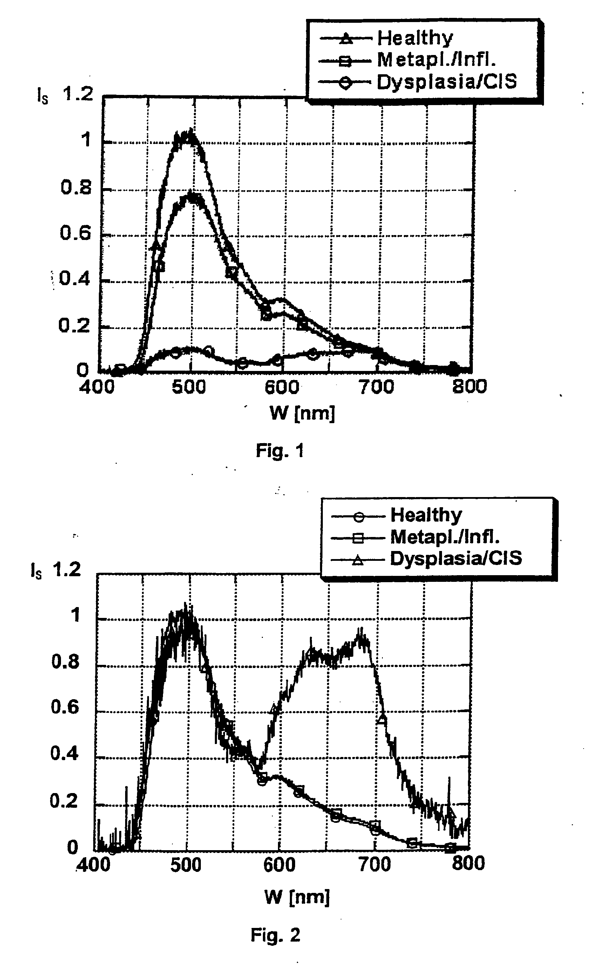

Zur Abhängigkeit der Fluoreszenzintensität vom Gewebezustand wird auf Fig. 1 hingewiesen: Dort ist beispielhaft für eine Anregungswellenlänge von 405 nm die spektrale Fluoreszenzintensität Iₛ über der Wellenlänge W in Nanometer für verschiedene Gewebezustände dargestellt (Healthy: gesund; Metapl./Infl.: Metaplasie/Entzündung, d. h. gutartige und damit als nicht maligne einzustufende Gewebeatypien; Dyspasia/CIS: Dysplasie/Carcinoma in situ). Aus Fig. 1 wird ersichtlich, dass mit zunehmendem Grad der Gewebeatypie bzw. mit zunehmendem Malignitätsgrad die integrale Intensität abnimmt.

Die Abhängigkeit der Fluoreszenzfarbe vom Gewebezustand, d. h. des beim Betrachter hervorgerufenen integralen Farbeindrucks, wird aus der Darstellung gemäß Fig. 2 ersichtlich: Hier sind die auf das erste Fluoreszenzmaximum (im grünen Spektralbereich, also um etwa 500 nm) normierten Kurvenverläufe der spektralen Fluoreszenzintensitäten Iₛ dargestellt. Der Unterschied zu der Darstellung gemäß Fig. 1 besteht lediglich in der Normierung zur Hervorhebung der Farbverschiebung. Die Kurven für die unterschiedlichen Gewebezustände sind jedoch als solche identisch mit denjenigen aus Fig. 1.

Wie zu erkennen ist, gilt, dass mit zunehmendem Grad der Gewebeatypie bzw. mit zunehmendem Malignitätsgrad der Anteil der Rotfluoreszenz (Wellenlängen zwischen etwa 600 nm und 700 nm) gegenüber der Grünfluoreszenz (Wellenlängen kleiner als etwa 570 nm) zunimmt und damit das Gewebe mit zunehmendem Grad der Gewebeatypie bzw. mit zunehmendem Malignitätsgrad für den Betrachter in zunehmendem Maße rötlich erscheint.

Die ausschließliche Bewertung der gewebezustandsabhängigen Fluoreszenzintensität für die Gewebedifferenzierung und damit für die Diagnose prä- und frühmaligner Läsionen, d. h. die Nichtbeachtung der vom Gewebezustand abhängigen integralen Farbwiedergabe bzw. Farbverschiebung, indem beispielsweise im Arbeitsmodus der Autofluoreszenzdiagnose der ohnehin intensitätsschwache rote Spektralbereich in die Gewebebewertung nicht miteinbezogen wird, ist problembehaftet, da als Ursache für einen Rückgang der Gesamtfluoreszenzintensität nicht ausschließlich Gewebeatypien in Betracht gezogen werden dürfen. Der Rückgang der Intensität kann auch gewebemorphologisch bedingt sein. Beispielsweise wirken sowohl mikroskopisch stark strukturiertes, aber gesundes Gewebe als auch makroskopische Oberflächenunebenheiten, wie evtl. Gewebefalten bei gesundem Gewebe, hinsichtlich des Fluoreszenz anregungslichts und auch bezüglich der Emission von Fluoreszenzlicht wie "Lichtfallen" und reduzieren dadurch das vom Auge oder von der Kamera detektierbare Fluoreszenzlicht. Damit wirkt dieses Gewebe u. U. ähnlich wie krankes Gewebe intensitätsschwächer.

Der Versuch der Gewebebewertung hinsichtlich prä- bzw. frühmaligner Läsionen allein auf der Basis der Fluoreszenzintensität kann deshalb vermehrt zu Falschpositiv-Resultaten führen und somit zu einer verminderten Spezifität. Unter diesem Aspekt spielt beim bildgebenden Autofluoreszenzdiagnoseverfahren für das Auffinden prä- bzw. frühmaligner Läsionen die vom Gewebezustand bzw. Malignitätsgrad abhängige Farbe des Gewebes und der so dem Betrachter vermittelte Farbeindruck eine entscheidende Rolle. Ein bildgebendes Fluoreszenzdiagnosesystem sollte daher so konzipiert sein, dass die vom Grad der Gewebeatypie abhängige Farbe, d. h. der Wechsel in der Gewichtung der spektralen Fluoreszenzanteile, dem Betrachter auch tatsächlich sichtbar gemacht wird.

Aus Fig. 1 wird allerdings auch ersichtlich, dass die integrale Fluoreszenzintensität von frühmalignem und malignem Gewebe gegenüber gesundem Gewebe ausserordentlich stark zurückgeht. In Verbindung mit der endlichen bzw. begrenzten Dynamik von konventionellen Farbkameras auch und des menschlichen Auges kann dann die Farbverschiebung gegenüber gesundem Gewebe unter Umständen kaum noch oder gar nicht mehr wahrgenommen werden; die verdächtige Stelle erscheint lediglich dunkel, und Farben sowie Farbverschiebungen sind dadurch nur eingeschränkt zu erkennen. Eine Helligkeitsanhebung führt lediglich dazu, dass das die verdächtige Stelle umgebende gesunde Gewebe überstrahlt wirkt und deshalb die Farbveränderungen besonders bei kleineren verdächtigen Stellen dann auch nicht wahrgenommen werden können.

Zu berücksichtigen ist außerdem, dass die gegenüber gesundem Gewebe stark reduzierte Fluoreszenz von prä- und frühmalignem Gewebe (siehe Fig. 1) dazu führt, dass die von der Kamera in diesen Gewebebereichen erzeugten Signale gegebenenfalls schon von einem vergleichsweise starken Rauschen überlagert sind, so dass eine Helligkeitsanhebung, beispielsweise in Form von elektronischer Verstärkung, ohnehin nur bedingt möglich ist.

Daraus ergibt sich eine Problematik, die vergleichbar mit derjenigen sein kann, welche bereits oben beschrieben wurde: Frühmaligne und maligne Läsionen können aufgrund ihrer stark reduzierten Fluoreszenzintensität und deshalb nur noch begrenzt wahrnehmbaren Farbverschiebung kaum von solchem gesunden Gewebe unterschieden werden, welches beispielsweise allein aufgrund einer veränderten Oberflächenstruktur sowohl hinsichtlich des Anregungslichts als auch bezüglich des dadurch erzeugten Fluoreszenzlichts "lichtschluckend" wirkt und welches deshalb gleichfalls weniger Fluoreszenzlicht als normal strukturiertes gesundes Gewebe dem Beobachter zuführen kann.

Unsicherheiten bei der Beurteilung solchen Gewebes macht in all solchen Fällen eine Probeentnahme obligatorisch, um möglichst alle potentiellen frühmalignen bzw. malignen Herde zu erfassen. Dadurch steigt aber die Falschpositiv-Rate, d. h. die Spezifität sinkt. In gleichem Maße steigt der Zeitaufwand; es entstehen dadurch bedingt zusätzliche Kosten, und es sind außerdem zusätzliche, gleichfalls zusätzliche Kosten verursachende Biopsien erforderlich. Außerdem kann jede Probeentnahme zu Blutungen führen, welche die weitere Untersuchung behindern.

Ungünstig beim Bestreben, in die Gewebedifferenzierung unter den gegebenen Umständen die Farbverschiebung miteinzubeziehen, ist, dass mit zunehmendem Malignitätsgrad bzw. mit zunehmender Atypie des Gewebes nicht nur die Fluoreszenz im grünen Spektralbereich rückläufig ist, sondern auch die Fluoreszenz im roten Spektralbereich - wenn auch weniger ausgeprägt (siehe Fig. 1). Aus mehrfacher Sicht wäre es aber vorteilhaft, wenn das detektierte Rotlicht bezüglich des Gewebezustands beispielsweise unverändert, d. h. konstant bliebe, wobei der dem Betrachter zugeführte Rotanteil zusätzlich idealerweise so hoch sein sollte, dass er einerseits von den durch die Grünfluoreszenz des gesunden Gewebes herrührenden Signalen klar dominiert wird, so dass also das gesunde Gewebe dem Betrachter grün erscheint, dass dieser Rotanteil aber andererseits die von der Grünfluoreszenz von krankem Gewebe herrührenden Signale, die etwa um einen Faktor 10 geringer sind als diejenigen, welche vom gesunden Gewebe stammen (siehe Fig. 1), deutlich übertrifft, so dass das kranke Gewebe dem Betrachter rot erscheint.

Ein vom Gewebezustand unabhängiger Rotanteil in der oben beschriebenen Art würde folgende Vorteile mit sich bringen: Es würde einerseits zu einer Verbesserung des Farbkontrasts zwischen gesundem und krankem Gewebe und damit zu einer Steigerung der Sensitivität führen, bedingt durch eine deutlich verbesserte Farbverschiebung zum Roten hin beim kranken Gewebe gegenüber dem gesunden Gewebe, und andererseits das schwach fluoreszierende kranke Gewebe heller erscheinen lassen, bedingt durch den dann höheren Rotanteil. Außerdem wäre eine bessere Differenzierung des kranken Gewebes auch von solchem gesunden Gewebe möglich, welches aufgrund einer mikroskopisch oder makroskopisch stärker strukturierten Oberflächenbeschaffenheit Licht verschluckt und deshalb dunkler erscheint als "glattes" gesundes Gewebe; letzteres erschiene dunkel, das frühmaligne und maligne Gewebe hingegen rot.

Da also die Rotfluoreszenz gemäß Fig. 1 eine nicht vernachlässigbare Abhängigkeit vom Grad der Gewebeatypie aufweist, nämlich mit zunehmender Gewebeveränderung wie die Grünfluoreszenz gleichfalls rückläufig ist, wenn auch weniger stark ausgeprägt, kann die Bereitstellung eines vom Gewebezustand unabhängigen Rotanteils nur über eine Bestrahlung des Gewebes mit zusätzlichem Rotlicht (neben dem Fluoreszenz anregungslicht) erfolgen; das dadurch vom Gewebe remittierte Rotlicht ist im Gegensatz zur Rotfluoreszenz vom Grad der Gewebeatypie weitgehend unabhängig. Gleichzeitig muß aber dafür gesorgt werden, dass die durch die Rotfluoreszenz bedingten Signalunterschiede bei unterschiedlichen Gewebezuständen gegenüber dem von der Rotremission erzeugten Signal im Rotkanal eliminiert werden oder zumindest nahezu bedeutungslos werden.

WO 00/42910 A1 beschreibt eine Vorrichtung zum Sichtbarmachen von Gewebeautofluoreszenz. Es geht davon aus, dass ein malignes Gewebe durch eine gegenüber gesundem Gewebe geringere Autofluoreszenz gekennzeichnet ist. Versucht wird, die Unterschiede hinsichtlich der Fluoreszenzintensität aufzuspüren und dadurch krankes Gewebe sichtbar zu machen. Dabei werden zwei eigenständige Gewebeabbildungen erzeugt, zum einen in Autofluoreszenzbild, bei dem das bei Bestrahlung mit ultraviolettem oder tiefviolettem sichtbaren Licht remittierte Fluoreszenzlicht verwendet wird, zum anderen ein Rotlicht, bei dem das bei Bestrahlung mit Rotlicht remittierte Rotlicht verwendet wird. Das Rotlichtbild wird nur als Referenzbild zu dem Autofluoreszenzbild benötigt. Das Autofluoreszenzbild und das Rotlichtbild werden digitalisiert und dann auf ihre Spitzenwerte normalisiert. Bildpunkt für Bildpunkt werden dann die Werte des Autofluoreszenzbildes durch die entsprechenden Werte des Rotlichtbildes geteilt. Das unterschreiten eines zuvor festgelegten Grenzwertes zeigt dann ein krankhaft verändertes Gewebe an. Das Referenzbild bzw. das Rotlichtbild wird in ein Schwarz-Weiß-Bild mit entsprechenden Grauschattierungen umgewandelt und das zuvor als krankhaft festgestellte Gewebe bzw. die zugehörigen Bildpunkte werden in diesem Schwarz-Weiß-Bild farbig markiert.

Aus der US 5 590 660 ist ein Diagnosesystem bekannt, bei dem von einer Lichtquelle bereitgestelltes und dann am Gewebe remittiertes Rotlicht in die Gewebebewertung eingeht, und zwar zusätzlich zu dem vom Gewebe emittierten Fluoreszenzlicht. Insofern ist die Konzeption des Systems und der Lichtquelle so geändert, dass eine verbesserte Farbdifferenzierung und damit eine verbesserte Sensitivität von prä- und frühmalignem Gewebe gegenüber derjenigen Vorgehensweise erreicht wird, bei welcher der Farbeindruck und damit der Gewebezustand nur auf der Basis der Autofluoreszenz des Gewebes bewertet werden. Allerdings ist dabei die Vorgehensweise derart, dass als Detektionseinheit *zwei* Kameras eingesetzt werden müssen, deren Sensoren optische Bandpaßfilter vorgelagert sind, welche Sonderanfertigungen darstellen in dem Sinne, dass sie nicht den Filterspezifikationen herkömmlicher, für die Weißlichtendoskopie geeigneter 3-Chip-Kameras entsprechen. Die der US 5 590 660 zu Grunde liegende Idee ist nämlich die, dass der gesamte detektierte Wellenlängenbereich aufgeteilt wird in zwei vollkommen separate spektrale Bereiche: Einen ersten Wellenlängenbereich, in welchem im Wesentlichen das gesamte Autofluoreszenzlicht liegt oder zumindest der weitaus größte Teil (Wellenlängen zwischen 500 nm und 650 nm, entsprechend der Empfindlichkeit der dort verwendeten Sensoren ist das jenseits von 650 nm detektierte Autofluoreszenzsignal verschwindend klein), das der ersten Kamera zugeführt wird, deren Signale einem ersten Farbeingang eines Monitors (z.B. Grün) zugeführt werden, und einen zweiten Wellenlängenbereich, welcher sich mit dem ersten Wellenlängenbereich nicht überschneidet und in welchem spektral das zusätzliche Beleuchtungslicht aus der Lichtquelle liegt (Rotlicht, Wellenlängen größer als 700 nm), welches nach Remission am Gewebe der zweiten Kamera zugeführt wird, deren Signale einem zweiten Farbeingang des Monitors (z. B. Rot) zugeführt werden.

Die in der US 5 590 660 beschriebene Vorgehensweise hat die Eigenschaft, dass das dem zweiten Farbkanal des Monitors zugeführte Signal vom Grad der Gewebeatypie unabhängig ist (mit den bezüglich der Farbdifferenzierung oben ausführlich beschriebenen Vorteilen), denn der Detektionsbereich der zweiten Kamera, die diesem zweiten Monitoreingang die Signale liefert, liegt außerhalb des Wellenlängenbereichs der emittierten Gewebeautofluoreszenzsignale, deren Intensität wiederum vom Gewebezustand abhängig sind. Dem bezüglich des Gewebezustands konstanten zweiten Signal kann sich also kein von der Autofluoreszenz herrührendes und damit gewebezustandsabhängiges Signal überlagern, und der zweite Kanal kann bei entsprechender Dosierung des zusätzlichen Beleuchtungslichts als Farbreferenz gegenüber den gewebezustandabhängigen Schwankungen der in den ersten Kanal eingespeisten Signale benutzt werden.

Andererseits ergibt sich aber bei der in der US 5 590 660 beschriebenen Vorgehensweise der Nachteil, dass die zu verwendenden Kameras bzw. deren Sensoren nicht Teil einer konventionellen und damit uneingeschränkt farbbildtauglichen (im Hinblick auf naturgetreue Farbe wiedergabe) 3-Chip-Kamera sein können, da der erste Detektionsbereich den Wellenlängenbereich des gesamten Autofluoreszenzlichts umfaßt, welches aus Grün-, aber auch aus Rotlicht besteht, während sich der zweite Detektionsbereich nicht mit diesem ersten Bereich überschneiden darf und dementsprechend erst im langwelligen roten Spektralbereich liegen kann. Eine konventionelle 3-Chip-Kamera jedoch trennt Rot und Grün bereits in einem wesentlich kurzwelligeren Bereich, und außerdem findet dort immer eine Überschneidung der den jeweiligen Sensoren zugeordneten Transmissionsbereiche statt. Soll also bei der Diagnosevorrichtung gemäß dieser Lösung eine Weißlichtdiagnose mit einer 3-Chip-Kamera durchgeführt werden, ist eine weitere konventionelle Farbkamera notwendig. Die Vorrichtung nach der US 5 590 660 ist dann entsprechend qufwändig aufgebaut und damit fürden Anwenderzum einen unhandlich und zum anderen teuer. Der Umstand, dass der der ersten Kamera zugeführte Wellenlängenbereich so breit ist bzw. so weit in den roten Spektralbereich reicht - was in Verbindung mit einem konventionellen Monitor in einem Falschfarbenbild resultiert -, wirkt sich auch insofern nachteilig aus, als dass sich die Fluoreszenzkurven für die unterschiedlichen Gewebezustände zum Langwelligen hin, insbesondere aber mit Beginn des gelben und roten Spektralbereiches immer mehr einander annähern und insofern - bei Einbezug auch dieser längerwelligen spektralen Bereiche - die Möglichkeit der Gewebedifferenzierung immer verwaschener und damit ungünstiger wird, wenn der der ersten Kamera zugeführte Spektralbereich bis ins Rote hineinreicht.

Des weiteren ist ein endoskopischer Direkteinblick, d. h. der Verzicht auf die Kameras im Modus der Autofluoreszenzdiagnose, nicht ohne weiteres möglich, da der Umfang des zusätzlich bereitgestellten Rotlichts auf die Falschfarbendarstellung der Spezialkamera angepaßt wurde.

Ferner ist aus der US 5 772 580 ein Diagnosesystem bekannt, das vollständig auf die Detektion von Rotlicht bei der Gewebebewertung verzichtet, d. h. sowohl auf die Rotfluoreszenz als auch auf zusätzliches von der Lichtquelle bereitgestelltes und am Gewebe remittiertes Rotlicht. Der Transmissionsbereich des dort zum Einsatz kommenden Sensors liegt zwischen 480 nm und 600 nm. Dadurch erzielt man zwar einerseits ebenfalls eine gesteigerte Sensitivität für prä- bzw. frühmaligne Läsionen, andererseits entstehen aber auch die oben erwähnten, mit dem Verzicht auf die Rotreferenz verbundenen Nachteile, vor allem die reduzierte Spezifität durch eine erhöhte Falschpositiv-Rate. Zwar ist mit der Vorrichtung gemäß dieser Schrift auch eine Weißlichtdiagnose möglich, jedoch ist hierfür eine zweite Kamera notwendig. Des weiteren bedeutet der Verzicht auf die Detektion von Rotlicht (sowohl Fluoreszenz als auch am Gewebe remittiertes Rotlicht) ein Verlust an Helligkeit. Deshalb verwendet das System auch große, schwere und unhandliche Bildverstärker, um dennoch ein ausreichend helles Bild zu erzeugen.

Bekannt sind auch noch Diagnosevorrichtungen, bei denen anstatt zusätzlichem, von der Lichtquelle emittiertem und am Gewebe remittiertem Rotlicht Blaulicht detektiert wird mit dem Ziel der verbesserten Farbkontrastierung des Gewebes im Diagnosemodus der Autofluoreszenz. Bei dieser Vorgehensweise wird ein geringer Anteil des von der Lichtquelle für die Fluoreszenzanregung zur Verfügung gestellten Blaulichts, welcher aber vom Gewebe nicht absorbiert, sondern am Gewebe remittiert wird, von der Kamera detektiert und nicht wie bei den anderen bekannten Systemen komplett im Bildpfad geblockt (beispielsweise durch optische Filter). Dabei ist der die Kamera erreichende Blauanteil mittels optischer Filtertechnik so spezifiziert und damit das Verhältnis von Grünfluoreszenz und remittiertem Blauanteil so festgelegt, dass gesundes Gewebe dem Betrachter grün erscheint, während krankes Gewebe bläulich wirkt. Die Beurteilung des Krankheitsgrades des Gewebes erfolgt also im Wesentlichen durch die Bewertung der unterschiedlichen, vom Gewebezustand abhängigen Grün-Blau-Anteile. Nachteilig bei dieser Vorgehensweise ist, dass im Gegensatz zu Rotlicht das kurzwelligere blaue Licht eine geringere Gewebeeindringtiefe hat. Dadurch ist beim vom Gewebe remittierten Blaulicht der gerichtet reflektierte Anteil größer als bei Rotlicht, der homogen zurückgestreute Anteil indes geringer als bei Rotlicht. Da das Gewebefluoreszenzlicht jedoch nahezu unabhängig vom Enfallswinkel des Anregungslichtes relativ homogen emittiert wird (isotrop), ist somit der beim Betrachter durch die Summe aus Fluoreszenz und vom Gewebe remittierten Blaulicht hervorgerufene Farbeindruck stark vom Einfallswinkel des Anregungs- und Beleuchtungslichtes abhängig, und zwar viel stärker als bei der Detektion von Fluoreszenzlicht und von vom Gewebe remittiertem Rotlicht. Die Gewebebeurteilung ist also vom Einfallswinkel des Beleuchtungs- und Anregungslichts abhängig, was natürlich keinesfalls erwünscht ist.

Diese Nachteile können mit einem System vermieden werden, das in der älteren deutschen Patentanmeldung 101 16 859.4 beschrieben ist und bei dem die Dämpfung von Rot elektronisch in der Kamera bzw. deren Controllervorgenommen wird. Allerdings erfordert diese Vorgehensweise eine Kamera, bei welcher beim Wechsel zwischen den Diagnosemethoden Kameraparameter verändert werden müssen (z. B. die Rotverstärkung). Dies verlangt zum einen, dass man eine separate Zugriffsmöglichkeit auf diese Parameter hat, und zum anderen, dass im Hinblick auf eine bequeme, einfache und vor allen Dingen schnelle Umschaltmöglichkeit diese Parameter über eine externe Steuereinheit elektronisch angesteuert und verändert werden können. Diese Anforderungen erlauben es nicht, auf eine beliebige, konventionelle endoskopische Kamera zurückzugreifen. Zumindest der Controller weicht insofern von einer herkömmlichen Bildverarbeitungseinheit ab. Der Anwender hat damit nicht die Möglichkeit, auf ein beliebiges Gerät, in dessen Besitz er vielleicht schon ist, zurückzugreifen. Es bestehen diesbezüglich keine Möglichkeiten, die Systemkosten zu reduzieren.

Der Erfindung liegt daher die Aufgabe zu Grunde, eine Diagnosevorrichtung zur bildgebenden Diagnose von Gewebe - insbesondere für die Untersuchung des Bronchialbereichs - unter wahlweiser Anwendung der bildgebenden Weißlichtdiagnose und der bildgebenden Autofluoreszenzdiagnose zu schaffen, mit der im Modus der bildgebenden Autofluoreszenzdiagnose einerseits eine gesteigerte Sensitivität, d. h. eine verbesserte Farbkontrastierung und damit eine verbesserte Unterscheidungsmöglichkeit zwischen gesundem und prä- bzw. frühmalignem Gewebe ermöglicht wird gegenüber jener Vorgehensweise, bei welcher im Modus der Autofluoreszenzdiagnose ausschließlich die Bewertung des Autofluoreszenzlichts selbst herangezogen wird, und andererseits eine gesteigerte Spezifität, d. h. eine verbesserte Unterscheidungsfähigkeit von prä- und frühmalignem Gewebe vorallen Dingen auch gegenüber solchem gesunden Gewebe, welches aufgrund seiner mikro- und / oder makrostrukturell unebenen und deshalb lichtschluckenden Oberfläche dem Empfänger vergleichsweise wenig Fluoreszenzlicht zuführt und deshalb fälschlicherweise als krankes Gewebe eingestuft werden könnte, ermöglicht wird.

Dabei soll zusätzlich der dem Betrachter im Autofluoreszenzmodus entstehende Farbeindruck des Gewebes vom Betrachtungswinkel, d. h. vom Winkel des Endoskops gegenüber der Gewebeoberfläche, weitestgehend unabhängig sein im Gegensatz zu derjenigen Vorgehensweise beispielsweise, bei welcher im Modus der Autofluoreszenzdiagnose für die Gewebebeurteilung zusätzlich am Gewebe remittiertes Blaulicht herangezogen wird.

Außerdem soll es im Gegensatz zu denjenigen bekannten Systemen, welche im Modus der Autofluoreszenzdiagnose ebenfalls zusätzlich vom Gewebe remittiertes Rotlicht für die Gewebebewertung einbeziehen, möglich sein, erstens die Mittel für die Bildaufnahme und Bildverarbeitung der Autofluoreszenzbilder uneingeschränkt auch für die konventionelle Weißlichtdiagnose verwenden zu können und zweitens dabei gleichzeitig sogar auf eine beliebige, evtl. bereits beim Anwender ohnehin zur Verfügung stehende endoskopische Kamera zurückgreifen zu können, d. h., es kann beispielsweise eine konventionelle Farbkamera Verwendung finden, deren Controller nicht in Kommunikation mit einer zentralen Systemsteuer einheit, welche insbesondere beim Wechsel zwischen Weißlichtmodus und Autofluoreszenzmodus aktiv wird, oder in Kommunikation mit anderen Systemkomponenten stehen muß und der somit keinerlei diagnosemethodegebundene (elektrische) Statusänderung beim Umschaltvorgang zwischen bildgebender Weißlichtdiagnose und bildgebender Autofluoreszenzdiagnose erfahren muß. Das gleiche gilt bei Verwendung eines Videoendoskops für dessen Controller. Ist also der Anwender bereits im Besitz einer Farbkamera oder eines Videoendoskopsystems, dann kann er auf dieses Gerät oder zumindest, was das Videoendoskopsystem betrifft, auf Komponenten davon zurückgreifen, und der Preis für das Gesamtsystem wird für ihn deutlich geringer.

Das System soll so aufgebaut sein, dass ein einfacher und schneller, d. h. nur an einen Tastendruck gebundener Wechsel zwischen bildgebender Weißlichtdiagnose und bildgebender Autofluoreszenzdiagnose realisiert werden kann. Im Modus der bildgebenden Weißlichtdiagnose darf die Bildqualität und dabei insbesondere die Farbwiedergabe, obwohl für beide Diagnosemethoden dieselbe Bildverarbeitungseinheit verwendet wird, keinerlei Einschränkungen erfahren. Und schließlich muss auch der direkte endoskopische Einblick, also der Verzicht auf Kamera oder Videoendoskop, mit den gleichen Vorzügen im Hinblick auf eine Sensitivitäts- und Spezifitätssteigerung möglich sein.

Zur Lösung dieser Aufgabe ist die eingangs erwähnte Vorrichtung dadurch gekennzeichnet, dass das Rotlicht so intensiv ist, dass das vom Gewebe remittierte Rotlicht die Gewebefluoreszenz dominiert, und dass das System im bildpfad optische Mittel aufweist, welche das Rotlicht so dämpfen, dass dieses quantitativ zwischen der relativ starken Grünfluoreszenz von gesundem Gewebe und der stark reduzierten Grünfluoreszenz von prä- bzw. frümalignem Gewebe liegt.

Hierdurch wird erreicht, dass der Rotanteil des vom Sensorsystem der Bildaufnahmeeinheit detektierten Lichts erstens vom Grad der Gewebeatypie praktisch unabhängig ist und als Referenz gegenüber dem vom Grad der Gewebeatypie sehr stark abhängigen Grünanteil herangezogen werden kann und zweitens quantitativ zwischen der relativ starken Grünfluoreszenz von gesundem Gewebe und der stark reduzierten Grünfluoreszenz von prä- bzw. frühmalignem Gewebe liegt, um so u. a. eine Verbesserung des Farbkontrastes zwischen benignem und prä- bzw. frühmalignem Gewebe zu realisieren. Mit diesen Maßnahmen kann sowohl die Sensitivität als auch die Spezifität der Diagnosevorrichtung gesteigert werden, wobei trotzdem ein einfacher apparativer Aufbau beibehalten werden kann und insbesondere die bildgebende Darstellung in beiden Arbeitsmoden mittels einer beliebigen, konventionellen endoskopischen Kamera oder eines Videoendoskops, dessen Controller in konventioneller Form aufgebaut ist, vorgenommen werden kann. Des weiteren kann aber - auch im Autofluoreszenzmodus und unter Beibehaltung der hier vorgestellten Vorzüge im Hinblick auf die Steigerung von Sensitivität und Spezifität - der endoskopische Direkteinblick vorgenommen werden, d. h. auf eine Kamera oder ein Videoendoskop verzichtet werden. Außerdem ist die Farbwiedergabe des Gewebes im Arbeitsmodus der bildgebenden Autofluoreszenzdiagnose vom Winkel zwischen der Gewebenormalen und dem Endoskop weitgehend unabhängig.

Wie bereits ausgeführt basiert der vom Grad der Gewebeatypie bzw. vom Malignitätsgrad abhängige und dem Betrachter vermittelte Farbeindruck des Gewebes im Modus der bildgebenden Autofluoreszenzdiagnose auf einem stark zunehmenden Rückgang der Grünfluoreszenz und einem weniger stark ausgeprägten, aber ungünstigerweise nicht vemachlässigbaren Rückgang der Roffluoreszenz mit zunehmender Atypie des Gewebes. Gesundes Gewebe erscheint dadurch grün, prä- bzw. frühmalignes Gewebe mit wachsender Atypie zunehmend rötlich bzw. rotbraun. Der Farbunterschied und damit die Unterscheidungsfähigkeit zwischen benignem und prä- bzw. frühmalignem Gewebe wäre allerdings ausgeprägter und deutlicher und damit die Sensitivität des Systems bezüglich prä- bzw. frühmalignen Läsionen erhöht, wenn der Rotanteil nicht ebenfalls mit zunehmender Gewebeatypie bzw. mit zunehmendem Malignitätsgrad abnehmen würde, sondern beispielsweise konstant oder zumindest nahezu konstant bliebe und dem Rotanteil somit eine Referenzfunktion gegenüber dem gewebezustandsabhängigen Grünanteil zukäme. Des weiteren wäre es im Hinblick auf die Farbdifferenzierung von Vorteil, wenn ausserdem der nun konstante Rotanteil gegenüber dem gewebezustandsabhängigen Grünanteil ohne eine Verschlechterung des Signal-Rausch-Verhältnisses gerade so hoch eingestellt werden könnte, dass gesundes Gewebe deutlich grün und (früh-) malignes Gewebe klar rot erschiene, mathematisch ausgedrückt also das Verhältnis von Grünfluoreszenz von gesundem Gewebe zu Rotanteil gleich dem Verhältnis von Rotanteil zu Grünanteil von krankem Gewebe wäre.

Dies wird bei der erfindungsgemäßen Vorrichtung u. a. dadurch erreicht, dass die Fluoreszenzanregungslichtquelle nicht nur das Fluoreszenzanregungslicht - bei der Autofluoreszenzanregung im Bronchialbereich in der Regel Violett-/Blaulicht - sondern auch noch zusätzlich Rotlicht zur Verfügung stellt und an das zu untersuchende Gewebe heranführt.

Da bei der erfindungsgemäßen Ausgestaltung eine vor allen Dingen auch bezüglich der optischen Eigenschaften des Kamerakopfes konventionelle Kamera oder aber ein bezüglich der optischen Eigenschaften der Sensoreinheit konventionelles Videoendoskop verwendet wird, um gleichermaßen auch eine konventionelle Weißlichtendoskokpie zu ermöglichen, findet - im Gegensatz zu der Vorgehensweise gemäß der US 5 590 660, deren Bildaufnahmeeinheit für den konventionellen Weißlichtbetrieb nicht taugt - in der Bildaufnahmeeinheit eine Überlagerung von vom Gewebe remittiertem und vom Grad der Gewebeatypie unabhängigem rotem Beleuchtungslicht und von vom Grad der Gewebeatypie abhängigem rotem Fluoreszenzlicht statt. Bei Verwendung konventioneller Kopf- bzw. Sensortechnik, welche auch einen hinsichtlich der Farbeigenschaften uneingeschränkten Weißlichtbetrieb zuläßt und insofern keine Falschfarbenbilder erzeugen darf, wie dies beispielsweise das System nach der US 5 590 660 tut, deren dem Sensorsystem vorgelagerten optischen Filter also auf die genormten Empfänger-Primärstrahler herkömmlicher Monitore abgestimmt sind [1], ist deshalb die Schaffung einer (durch zusätzlich von der Lichtquelle bereitgestelltem und am Gewebe remittierten Rotlicht) Rotreferenz, welche quantitativ zwischen dem Grünanteil von gesundem Gewebe und dem Grünanteil von (früh-) maligenem Gewebe liegt, zunächst nicht möglich, da eine Eliminierung oder gar Ausschaltung des von der Gewebeatypie abhängigen roten Fluoreszenzlichts bei gleichzeitiger Etablierung eines von der Lichtquelle bereitgestellten Konstantrotanteils nicht realisierbar ist.

Die Problematik wird nachfolgend am Beispiel einer konventionellen, uneingeschränkt weißlichttauglichen 3-Chip-Kamera erläutert, gilt aber im Prinzip genauso für andere Kameras und Videoendoskopsysteme:
Bei der konventionellen 3-Chip-Kamera wird das im Kamerakopf eintreffende Licht mittels dichroitischer Filter aufgeteilt in "Blaulicht", "Grünlicht" und "Rotlicht" und dann unterschiedlichen Sensoren zugeführt. Da bei der hier betrachteten Autofluoreszenzanregung praktisch kein Blaulicht induziert wird, wird der Blaulichtpfad in diesem Arbeitsmodus nachfolgend nicht weiter betrachtet. Zunächst jedoch problematisch für die beschriebene Vorgehensweise bei der Autofluoreszenzdiagnose ist, dass der "Rotsensor" einer 3-Chip-Kamera mit konventionellem und damit weißlichtdiagnosetauglichem Kamerakopf bzw. das dem "Rotsensor" vorgelagerte optische Filter so spezifiziert ist, dass dieses nicht nur das von der Lichtquelle zusätzlich bereitgestellte, vom Gewebe remittierte und vom Grad der Gewebeatypie unabhängige rote Beleuchtungslicht detektiert, um so die oben angesprochene Rotreferenz realisieren zu können, sondern gleichzeitig auch das vom Malignitätsgrad abhängige rote Fluoreszenzlicht, denn der Transmissionsbereich des dem "Rotsensor" vorgelagerten optischen Filters, welches den spektralen Detektionsbereich des "Rotsensors" bestimmt, beginnt üblicherweise bereits bei Wellenlängen, die deutlich kleiner als 600 nm sind. Bei diesen Wellenlängen ist die Autofluoreszenz des Gewebes allerdings noch keinesfalls vernachlässigbar gering, und das im Rotkanal der Kamera verarbeitete und dem Monitor zu Verfügung gestellte Signal, welches also als Summe aus Fluoreszenzsignal und Remissionssignal betrachtet werden kann, unterliegt deshalb gewebezustandsbedingten Schwankungen, welche auf die malignitätsgradabhängigen Schwankungen des Fluoreszenzlichts zurückzuführen sind.

Soll dennoch trotz Verwendung einer konventionellen 3-Chip-Kamera ein vom Grad der Atypie des Gewebes praktisch unabhängiges Rotsignal realisiert werden, dann ist der von der Lichtquelle im roten Spektralbereich bereitgestellte Anteil so hoch zu bemessen, dass das vom Gewebe remittierte Rotlicht, dessen Intensität unabhängig vom Malignitätsgrad des Gewebes ist, das rote und vom Malignitätsgrad des Gewebes abhängige Fluoreszenzlicht um ein Vielfaches dominiert; d. h. beispielsweise bei der Verwendung einer breitbandigen Weißlichtquelle ist derTransmissionsgrad des Fluoreszenzanregungsfilters in der Lichtquelle im roten Spektralbereich so hoch zu wählen, dass das Rotfluoreszenzlicht gegenüber dem von der Lichtquelle stammenden und am Gewebe remittierten Rotlicht keine oder nur eine vernachlässigbare Rolle spielt. Je größer der Anteil des Remissionslichts gegenüber dem Fluoreszenzlicht am gesamten von der Kamera detektierten Rotlicht ist, um so größer ist die Unabhängigkeit des dem Monitor zugeführten Rotkanalsignals vom Grad der Gewebeatypie und damit um so besser die Möglichkeit der Differenzierung von prä- bzw. frühmalignem Gewebe von gesundem Gewebe (stärkere Rotverschiebung beim früh- und malignen Gewebe gegenüber dem benignen Gewebe).

Gleichzeitig muß aber in einem zweiten Schritt im Diagnosemodus der bildgebenden Autofluoreszenzdiagnose das Licht oder das Signal, welches seinen Ursprung im roten Spektralbereich hat, gedämpft werden, und zwar entsprechend der Intensität des zusätzlichen Rotlichts beziehungsweise entsprechend dem Verhältnis des Fluoreszenzanregungslichts (im Blauen / Violetten) zum zusätzlichen Rotlicht. Die hohe Intensität des zusätzlichen Rotlichts, die idealerweise so hoch gewählt ist, dass die Rotfluoreszenz gegenüber der Rotremission am Gewebe vernachlässigbar oder nahezu vernachlässigbar wird, und die dementsprechend starke Rotremission am Gewebe würde nämlich sonst - ohne Dämpfung des Rotkanalsignals - die Grünfluoreszenz sowohl von krankem, u. U. aber auch von gesundem Gewebe dominieren, und das Gewebe erschiene somit durchweg rot oder zumindest rötlich, und zwar sowohl das kranke als auch das gesunde. Es gäbe also fast keine oder eine zumindest verschlechterte Unterscheidungsmöglichkeit anhand des vermittelten Farbeindrucks.

Die Dämpfung des Rotsignals im Modus der bildgebenden Autofluoreszenzdiagnose wird daher derart vorgenommen, dass die Überlagerung von Gewebefluoreszenz und von der Lichtquelle zusätzlich bereitgestelltem und vom Gewebe remittiertem Rotlicht dazu führt, dass gesundes Gewebe grün erscheint, was gleichbedeutend ist mit einer Dominanz der Grünfluoreszenz gegenüber der gewebezustandsunabhängigen Rotremission - die Rotfluoreszenz spielt nun fast keine Rolle mehr - und prä- bzw. frühmalignes Gewebe rot erscheint, was einer Dominanz der gewebezustandsunabhängigen Rotremission gegenüber der im Vergleich zu gesundem Gewebe bei krankem Gewebe stark reduzierten Grünfluoreszenz gleichkommt. Quantitativ liegt also das im Wesentlichen von durch am Gewebe remittiertem Rotlicht bestimmte Rotkanalsignal nach der Dämpfung zwischen dem Grünkanalsignal, welches von Grünfluoreszenz licht von gesundem Gewebe erzeugt wird, und dem Grünkanalsignal, welches von Grünfluoreszenzlicht von krankem Gewebe erzeugt wird.

Bei der in der vorerwähnten älteren deutschen Patentanmeldung beschriebene Vorrichtung wird die Dämpfung von Rot elektronisch in der Kamera bzw. deren Controller vorgenommen. Diese Vorgehensweise ist jedoch mit mehreren Nachteilen verbunden:
Eine elektronische Dämpfung des Rotkanalsignals erfordert eine Zugriffsmöglichkeit auf einen speziellen Teil der Kameraelektronik, welche über die von außen zugänglichen Bedienelemente einer konventionellen endoskopischen Kamera gewöhnlicherweise nicht vorliegt. Außerdem muss diese elektronische Rotdämpfung im Modus der Weißlichtdiagnose rückgängig gemacht werden, um eine Cyanstichigkeit des Weißlichtbildes zu vermeiden. Wird dann wieder zurückgeschaltet in den Autofluoreszenzmodus, muss die elektronische Rotdämpfung wiederzugeschaltet werden. Soll diese Ab- und Zuschaltung zum Zweck einer bequemen Handhabung des Systems automatisch erfolgen und nicht manuell ausgeführt werden müssen, ist es weiterhin erforderlich, dass die Kamera mit einer zentralen Steuereinheit kommunizieren kann, über welche die Umschaltvorgänge zwischen den beiden Arbeitsmoden beispielsweise über Tastendruck zentral koordiniert werden und welche die entsprechenden diagnosemodusgebundenen Statussignale zentral vergibt. Diese beiden Anforderungen an die Kamera bzw. deren Controller erlauben es nicht, eine beliebige endoskopische Kamera, die der Anwender beispielsweise vielleicht ohnehin schon besitzt, einzusetzen. Das Gesamtsystem wird dadurch aufwändiger und potentielle Systempreisvorteile durch Verwendung einer bereits vorhandenen Kamera sind damit ausgeschlossen.

Sehr schwer wiegt aber bei der Rotdämpfung auf elektronischem Wege vor allen Dingen auch der Nachteil, dass beim Versuch, die durch die Abhängigkeit der Rotfluoreszenz vom Grad der Gewebeatyppie ausgelösten Schwankungen der Rotsignale durch einen sehr hohen Anteil von zusätzlich von der Lichtquelle bereitgestelltem Rotlicht idealerweise nahezu vollständig zu eliminieren und so den gewünschten konstanten, d. h. vom Grad der Gewebeatypie unabhängigen Rotanteil zu generieren (Rotreferenz), die Kamera leicht in Sättigung gehen kann, ohne dass sich dies dem Anwender wie sonst üblich über zu helle Monitorbilder andeuten würde. Auch dieses Verhalten kann am Beispiel der 3-Chip-Kamera gut erläutert werden, gilt aber prinzipiell auch für andere Bildaufnahmeeinheiten: Bei der elektronischen Rotkanalsignaldämpfung erfolgt die Dämpfung und damit auch die Reduktion der am Monitor vermittelten Helligkeitsverhältnisse erst, nachdem bereits das Rotlicht vom "Rotsensor" der 3-Chip-Kamera detektiert wurde und ein elektronisches Signal im Sensor erzeugt wurde. Die Höhe der vorgenommenen Dämpfung orientiert sich, wie das oben bereits erläutert wurde, am Grünfluoreszenzsignal, d. h., die Intensität des am Monitorwahrnehmbaren Rotlichts liegt - unabhängig zu dem am "Rotsensor" eintreffenden Umfang an Rotlicht - immer in der Größenordnung des Grünfluoreszenzlichts, idealerweise ungefähr beim Mittelwert von der Grünfluoreszenz von gesundem Gewebe und der Grünfluoreszenz von krankem Gewebe, was u. U. aber völlig im Gegensatz zur Intensität des tatsächlich vom "Rotsensor" delektierten Rotlichts gegenüber dem vom "Grünsensor" detektierten Grünlicht ist. Helligkeitsregelmechanismen, wie beispielsweise eine vom Anwender vorgenommene Abstandsreduktion der Endoskopspitze zum Gewebe zur Steigerung der Bildhelligkeit im Autofluoreszenzmodus, werden aber durch die vom Monitorvermittelten scheinbaren Helligkeitsverhältnisse bestimmt, welche aber nicht den tatsächlichen Helligkeitsverhältnissen an den Sensoren entsprechen, da den "Rotsensor" tatsächlich wesentlich mehr Licht erreicht. Wird also beispielsweise der Abstand Endoskopspitze - Gewebe so weit reduziert, dass die intensitätsschwache Autofluoreszenz zu einem ausreichend hellen Bild führt, dann ist - bei entsprechend hoch gewähltem Anteil von zusätzlich von der Lichtquelle bereitgestelltem Rotlicht zur Schaffung einer echten Rotreferenz- das tatsächlich am "Rotsensor" eintreffende Rotlicht wesentlich stärker als dies der Monitor dem Betrachter vermittelt. Entsprechend liefert der Monitor ein Bild mit gewünschter Bildhelligkeit, während sich aber der "Rotsensor" schon längst in Sättigung befindet. Letzteres führt aber dazu, dass das von der Lichtquelle zusätzlich bereitgestellte und am Gewebe remittierte Rotlicht nicht mehr seiner Referenzfunktion gerecht wird.

Dieses Problem kann nur gelöst werden, indem man durch Limitierung des zusätzlich von der Lichtquelle bereitgestellten Rotlichts dafür sorgt, dass das vom Gewebe remittierte Rotlicht nicht oder nur geringfügig intensiver ist als das induzierte Autofluoreszenzlicht von gesundem Gewebe. Eine Limitierung des zusätzlich von der Lichtquelle bereitgestellten Rotlichts bedeutet aber andererseits eine nur eingeschränkte Möglichkeit der Eliminierung der unerwünschten Rotschwankungen, welche durch die vom Grad der Gewebeatypie abhängige Rotfluoreszenz verursacht werden; dem Ziel der Etablierung einer vom Grad der Gewebeatypie unabhängigen echten Rotreferenz kann man sich dementsprechend nur mehr oder weniger gut nähern, man kann es aber nicht tatsächlich realisieren.

Eine Vermeidung der oben geschilderten Probleme bei gleichzeitigem Erhalt der Vorteile, die dieses Verfahren mit sich bringt, vor allen Dingen die Verbesserung von Sensitivität und Spezifität gegenüber der alleinigen Bewertung der Gewebefluoreszenz kann dadurch erreicht werden, dass statt der elektronischen Dämpfung des Rotkanalsignals erst in der Bildverarbeitungseinheit eine optische Dämpfung des Lichts, welches dem roten Spektralbereich zuzuordnen ist, erfindungsgemäß bereits in der Bildüber tragungseinheit und / oder in der Bildaufnahmeeinheit vorgenommen wird. Das bedeutet, dass eine Dämpfung von "Rot" bereits vorgenommen wird, bevor es überhaupt erst das Sensorsystem erreicht mit den nachfolgenden, vorteilhaften Konsequenzen:
Eine unerwünschte Sättigung des Sensorsystems in der oben beschriebenen Form, also eine Sättigung, welche durch die absichtliche Bereitstellung eines vergleichsweise hohen Quantums an zusätzlichem Rotlicht ausgelöst wird, obwohl das Monitorbild dabei keinesfalls überstrahlt wirkt (bedingt durch die im Controller vorgenommenen elektronische Dämpfung), ist nicht mehr möglich, da das Rotlicht der Lichtquelle in seinem vollem Umfang das Sensorsystem nun gar nicht mehr erreichen kann, sondern eben nur noch in gedämpfter Form.

Durch die Transformation der elektronischen Dämpfung in eine optische Dämpfung ist es nicht länger notwendig, beim Umschalten zwischen den beiden Arbeitsmoden (Weißlichtdiagnose und Autofluoreszenzdiagnose) in die Elektronik der Bildverarbeitungseinheit einzugreifen.

Durch die Verlagerung der Rotdämpfung in einen Bereich außerhalb der Bildverarbeitungseinheit und durch die zumindest partielle Unterbringung der Rotdämpfung in den Bereich der Bildübertragungseinheit ist es nicht mehr erforderlich, die Kamera oder das Videoendoskopsystem mit einer zentralen Steuereinheit kommunizieren zu lassen, welche diagnosemodusgebundene Statusänderungen an den Bildverarbeitungseinheiten vomehmen würde.

Nur so wird es möglich, auf eine beliebige konventionelle Kamera zurückzugreifen und mit dieser in einfachster Weise sowohl eine Autofluoreszenzdiagnose als auch eine Weißlichtdiagnose durchführen zu können. Keine andere derzeit bekannte Vorgehensweise oder gar bereits verfügbares System bietet die Möglichkeit, eine beliebige konventionelle Kamera in das System zu integrieren und dabei noch alle die oben genannten Vorteile umzusetzen, vor allen Dingen eine verbesserte Sensitivität und Spezifität. Wird statt der Kamera ein Videoendoskopsystem verwendet, dann kann zumindest auf einen gewöhnlichen Videoendoskop-Controller, u. U. aber sogar auch auf das konventionelle Videoendoskop selbst zurückgegriffen werden.

Durch die Transformation der elektronischen Dämpfung in eine optische Dämpfung ist es außerdem in einfacher Weise möglich, einen Endoskopdirekteinblick zu realisieren, also auf Kamera oder Videoendoskop zu verzichten, ohne dabei die genannten Vorzüge hinsichtlich der gesteigerten Sensitivität und Spezifität aufgeben zu müssen. Es muß lediglich dafür gesorgt werden, dass die Mittel zur optischen Dämpfung im Bildpfad, also in diesem Fall zwischen zu untersuchendem Gewebe und menschlichem Auge, erhalten bleiben. Die Beleuchtungs- bzw. Anregungsseite erfährt keine Veränderungen.

Dieses Vorgehen führt in vorteilhafter Weise zu einer deutlichen Steigerung der Sensitivität für prä- bzw. frühmalignes Gewebe gegenüber dem Fall, dass im roten Spektralbereich nur Fluoreszenzlicht und kein von der beleuchtenden und Fluoreszenz anregenden Lichtquelle zusätzlich emittiertes und am Gewebe remittiertes Rotlicht detektiert wird, welches anschließend im Bildpfad in der oben beschriebenen Form weiter verarbeitet wird.

Der von der Bildaufnahmeeinheit delektierte Rotanteil ist nun nicht mehr oder zumindest kaum noch vom Malignitätsgrad des Gewebes abhängig und nimmt deshalb nicht mehr oder kaum noch mit zunehmendem Malignitätsgrad ab. Krankes Gewebe erfährt dadurch einerseits eine deutlichere Rotverschiebung und strahlt andererseits bei entsprechender Einstellung der Rotdämpfung kräftiger, da die Rotdämpfung idealerweise so vorgenommen wird, dass der vom Gewebe remittierte, hinsichtlich der Gewebeatypie konstante Rotanteil ein deutlich stärkeres Rotsignal erzeugt, als die reduzierte Rotfluoreszenz beim kranken Gewebe, so dass also nicht nur das kranke Gewebe eine stärkere Rotstichigkeit erhält, sondern dass es auch heller erscheint und damit besser wahrgenommen werden kann, als wenn ausschließlich Fluoreszenzlicht betrachtet wird.

Für die Ausgestaltung der Lichtquelle bestehen verschiedene Möglichkeiten. Gemäß einer ersten Ausführungsform ist vorgesehen, dass die Lichtquelle aus einer Weißlichtquelle, d. h. einer breitbandig über den gesamten sichtbaren Spektralbereich emittierenden Lampe besteht, die ihr Licht zumindest zeitweise, nämlich im Modus der Autofluoreszenzdiagnose, über einen optischen Filter abgibt, der eine Lichttransmission im blauen / violetten Spektralbereich und in einem Spektralbereich im Roten, welcher idealerweise am langwelligen Ende des Sichtbaren liegt, erlaubt. Alternativ ist es möglich, dass die Lichtquelle aus einer blaulichtgefilterten Weißlichtquelle und einerzusätzlichen Rotlichtquelle besteht. Diese zusätzliche Rotlichtquelle kann beispielsweise ein Rotlichtlaser, z. B. ein Halbleiterlaser, oder auch eine rote LED oder ein Array von roten LED's sein. Weiterhin kann die Lichtquelle auch aus einem Blau-/Violettlichtlaser und aus einem Rotlichtlaser bestehen. In diesem Falle kann zwecks Durchführung der Weißlichtdiagnose außerdem ein Grünlichtlaser vorgesehen werden, der zusammen mit dem blauen und roten Laser das Weißlicht erzeugt. Um eine gegenüber der Version mit den Lasern verbesserte Farbwiedergabe im Weißlichtmodus zu realisieren, kann auch ein Array von relativ breitbandig, in unterschiedlichen spektralen Bereichen emittierenden Leuchtdioden verwendet werden, welche in der Summe idealerweise den gesamten sichtbaren Spektralbereich abdecken.

Abhängig von den getroffenen Maßnahmen zur optischen Rotdämpfung im Bildpfad kann es passieren, dass das Weißlichtbild cyanstichig erscheint, da die Mittel zur optischen Rotdämpfung aus Gründen der bequemen Handhabung des Systems im Sinne eines einfachen und schnellen Wechsels zwischen den beiden Diagnosemethoden auch im Weißlichtmodus an ihrem Platz im Bildpfad bleiben können sollen. Wird also beispielsweise im Okular des Endoskops ein zusätzliches optisches Filterplättchen untergebracht mit einer Beschichtung, welche im blauen und grünen Spektralbereich eine nahezu hundertprozentige Transmission ermöglicht, während die Transmission im Roten auf einen Bruchteil reduziert ist, dann fehlen diese Rotanteile bei der Bildwiedergabe im Modus der Weißlichtdiagnose. Dies könnte theoretisch durch einen Weißabgleich korrigiert werden, bei welchem in der Bildverarbeitungseinheit eine kompensatorische, elektronische Rotsignalverstärkung durchgeführt würde. Da jedoch eine beliebige konventionelle Kamera nicht in Kommunikation mit einer zentralen Steuereinheit, welche die Modenumschaltvorgänge koordiniert, treten kann - eine beliebige Kamera ist nicht für einen Signalaustausch mit dieser Steuereinheit konzipiert - und diese beim Weißabgleich hervorgerufene Rotsignalverstärkung deshalb auch beim Umschalten in den Modus der Autofluoreszenzdiagnose beibehalten würde, fände auch dort, also im Modus der Autofluoreszenzdiagnose, eine elektronische Kompensation der dort ursprünglich absichtlich herbeigeführten optischen Rotdämpfung statt.

Will man also die absichtlich herbeigeführte optische Rotdämpfung im Modus der Autofluoreszenzdiagnose beibehalten und dennoch im Weißlichtmodus ein Bild ohne Farbstichigkeit generieren, dann muß in den Strahlengang der Lichtquelle beim Umschalten in den Modus der Weißlichtdiagriose ein optisches Filter eingeschwenkt werden, welches zu oben beispielhaft angeführtem optischen Filterplättchen im Okular des Endoskops als Mittel der optischen Rotdämpfung optisch komplementär ist. Optisch komplementär bedeutet, dass die Überlagerung des sich im Okular in allen Moden fest befindlichen Filterplättchens mit demjenigen, welches beim Umschalten in den Weißlichtmodus in den Strahlengang der Lichtquelle eingeschwenkt wird, in einer idealerweise konstanten Transmission über den gesamten sichtbaren Spektralbereich resultiert. Das im Weißlichtmodus in den Strahlengang der Lichtquelle eingeschwenkte Filterplättchen hätte also im roten Spektralbereich eine nahezu hundertprozentige Transmission und eine Transmission im Blauen und Grünen, welche auf einen Wert reduziert ist, welcher der Rottransmission des Filterplättchens im Okular des Endoskops entspricht.

Die Umschaltvorgänge zwischen dem Arbeitsmodus der Weißlichtdiagnose und der Autofluoreszenzdiagnose werden von einer zentralen Steuereinheit koordiniert, die im Hinblick auf eine gute Systemkompaktheit zweckmäßigerweise in der Lichtquelle untergebracht ist und über Tastendruck angesteuert werden kann. Diese zentrale Steuereinheit sorgt dafür, dass im Modus der Autofluoreszenzdiagnose das Fluoreszenzanregungsfilter mit der zusätzlichen Rottransmission, wenn man auf zusätzliche Rotlichtquellen verzichten will, in den Strahlengang der Lichtquelle eingeschwenkt ist und dass im Modus der konventionellen Weißlichtdiagnose, sofern erforderlich (dies ist abhängig von den Mitteln der optischen Rotdämpfung), das Filter mit der reduzierten Blau- und Grüntransmission in den Strahlengang der Lichtquelle eingeschwenkt ist. Durch die hier vorgestellte Vorgehensweise gelingt es also, die durch den Wechsel zwischen den Arbeitsmoden erforderlichen Systemparameterveränderungen auf Komponenten in der Lichtquelle zu konzentrieren und damit weitere Geräte und insbesondere die Kamera von diesen arbeitsmodusbedingten Veränderungen auszuschließen. Nur so ist es möglich, beispielsweise eine beliebige konventionelle Kamera, deren Remote-Konzept in der Regel nicht auf ein bestimmtes System für die kombinierte Autofluoreszenz- und Weißlichtdiagnose abgestimmt ist, dennoch in das System zu integrieren.

Wie bei der Lichtquelle bestehen auch bei der Ausgestaltung der Bildübertragungseinheit verschiedene Möglichkeiten. Als Bildübertragungseinheit wird hierzunächst ganz allgemein diejenige Ansammlung von Komponenten verstanden, welche für die Abbildung des zu untersuchenden Objekts auf das Sensorsystem der Bildaufnahmeeinheit verantwortlich ist. Wählt man als Bildaufnahme- und Bildverarbeitungseinheit beispielsweise eine Kamera, besteht die Bildübertragungseinheit in der Regel zunächst aus einem Endoskopobjektiv, welches das zu untersuchende Objekt, beispielsweise Bronchialschleimhaut, auf die Stirnfläche eines Bildfaserbündels oder den Eingang eines mehrteiligen (Stab-) Linsensystems abbildet. Von dort findet gewöhnlich eine Bildübertragung auf das Okularlinsensystem des Endoskops statt. Das abschließende Element der Bildübertragungseinheit bildet in der Regel das Kameraobjektiv, welches das vom Okular erzeugte virtuelle Zwischenbild auf das Sensorsystem der Bildaufnahmeeinheit abbildet.

Verwendet man statt der Kamera ein Videoendoskop, besteht die Bildübertragungseinheit lediglich aus einem Objektiv, welches das zu untersuchende Objekt direkt auf das Sensorsystem des Videoendoskops abbildet.

Für die Realisierung der optischen Rotdämpfung gibt es diverse Möglichkeiten. Sämtliche oben genannten optischen Komponenten des Bildpfades können beispielsweise mit einer Beschichtung versehen werden, welche eine nahezu hundertprozentige Transmission im Kurzwelligen und eine reduzierte Transmission im Roten erzeugt. Es können aber für die optischen Komponenten des Bildpfades Materialien verwendet werden mit einer reduzierten Transmission im roten Spektralbereich. Denkt man zum Beispiel an die Verwendung eines Fiberendoskops, dann könnte eine Bildfaser mit reduzierter Rottransmission Verwendung finden. In diesem Fall kann auf eine zusätzliche, rotdämpfende Beschichtung der optischen Komponenten des Bildpfades verzichtet werden. Eine weitere Möglichkeit besteht im Einbringen eines optischen Filterplättchens mit den entsprechenden optischen Spezifikationen in den Bildpfad. Dessen Positionierung ist an den unterschiedlichsten Orten denkbar, beispielsweise wie im obigen Beispiel im Okular des Endoskops oder auch im oder am Kameraobjektiv.

Eine zumindest partielle Dämpfung von Licht, welches dem roten Spektralbereich zuzuordnen ist, kann aber auch schon durch den Einsatz einer entsprechenden Bildaufnahmeeinheit erzielt werden.

Alle bekannten Systeme für die bildgebende Darstellung der Autofluoreszenz verwenden spezielle Kameras. Fast alle verwenden Bildaufnahmeeinheiten mit einer maximalen Transmission im Spektralbereich der Autofluoreszenz und dabei insbesondere mit einer maximalen Transmission auch im Roten. Diese Vorgehensweise hat vor allen Dingen zwei Vorteile: Da die Gewebefluoreszenz nur sehr schwach ist, kann mit der bestmöglichen Detektion des Fluoreszenzlichts, d. h. mit der Detektion von idealerweise dem gesamten erzeugten und vom Bildpfad eingefangenen Fluoreszenzlicht, mit vergleichsweise bescheidenem Aufwand doch noch ein ausreichend helles Monitorbild erzeugt werden. Außerdem kann die spektrale Gewichtsverschiebung weg von der Grünfluoreszenz hin zur Rotfluoreszenz und damit die Farbverschiebung zum Roten mit zunehmender Atypie des Gewebes nur dann optimal dargestellt werden, wenn auch tatsächlich das gesamte Rotfluoreszenzlicht am Sensorsystem der Bildaufnahmeeinheit ankommt und nicht bereits im Bildpfad partiell geblockt wird. Ansonsten erschiene atypisches Gewebe nicht in einem rötlichen Farbton, sondern lediglich abgedunkelt, aber u. U. immer noch von der Grünfluoreszenz dominiert, und könnte so mit solchem gesunden Gewebe verwechselt werden, welches beispielsweise aufgrund seiner lichtschluckenden Oberflächenstruktur gleichfalls abgedunkelt erscheint. Eine die Rotdämpfung kompensierende elektronische Rotverstärkung käme einer Verschlechterung des Signal-Rausch-Verhältnisses gleich, was bei den ohnehin geringen Rotfluoreszenzen kaum akzeptiert werden kann.

Im Gegensatz dazu sind konventionelle, ursprünglich für die Weißlichtendoskopie konzipierte und im Hinblick auf eine ideale Farbwiedergabe optimierte Bildaufnahmeeinheiten, also beispielsweise der Kopf einer konventionellen 3-Chip-Kamera, hinsichtlich ihrer spektralen Detektion auf die (genormten) Empfänger-Primärstrahler angepaßt [1], d. h., die den Sensoren dieser Bildaufnahmeeinheiten vorgelagerten optischen Filter erzeugen im gelben und roten Spektralbereich eine reduzierte Transmission und damit eine optische Dämpfung, welche mit ansteigender Wellenlänge sogar zunimmt. Dadurch wird also allein schon durch den Einsatz einer konventionellen, für eine optimale Farbwiedergabe bei der Weißlichtendoskopie konzipierten Bildaufnahmeeinheit eine wenn auch noch nicht optimale, so doch zumindest partielle Rotdämpfung erzielt. Die unerwünschten und durch die Abhängigkeit der Rotfluoreszenz vom Grad der Gewebeatypie verursachten Schwankungen des vom Monitor wiedergegebenen Rotlichts werden dadurch bereits merklich reduziert.

Insofern ist eine gewöhnliche für die Weißlichtendoskopie konzipierte Bildaufnahmeeinheit gegenüber einer solchen, welche nahezu das gesamte Rotlicht detektieren kann, zu bevorzugen: Neben dem uneingeschränkt guten Weißlichtbild liefert sie bereits einen Beitrag zu der hier angestrebten Vorgehensweise bei der Steigerung von Sensitivität und Spezifität durch Etablierung einer Rotreferenz.

Bei der Bildaufnahmeeinheit handelt es sich damit entweder um einen gewöhnlichen Kamerakopf, beispielsweise dem Kopf einer 1-Chip- oder 3-Chip-Kamera, oder um das gewöhnliche Sensorsystem eines Videoendoskops. Die Bildverarbeitungseinheit wird durch den jeweils zugehörigen Controller repräsentiert. Auch hierbei handelt es sich um eine in jeglicher Sicht konventionelle Komponente: Da bei der hier beschriebenen Vorgehensweise das Problem der Rotdämpfung erstens optisch gelöst wird und zweitens außer den gerade beschriebenen und in der Bildaufnahmeeinheit vorgenommenen Maßnahmen (Anpassung der dort sich befindlichen optischen Filter an die Empfänger-Primärstrahler) die Mittel zur Rotdämpfung in die Bildübertragungseinheit verlegt werden, entfällt die Notwendigkeit der Zugriffsmöglichkeit auf den Controller beim Umschalten zwischen den beiden Arbeitsmoden, es existieren also keinerlei speziellen Anforderungen an diese Komponente.

Weitere vorteilhafte Merkmale der Vorrichtung sind in den Unteransprüchen angegeben und werden auch anhand eines in der Zeichnung dargestellten Ausführungsbeispiels beschrieben. In der Zeichnung zeigen:
- Fig. 1: den Verlauf der spektralen Fluoreszenzintensität Iₛ (willkürliche Enheiten) von Gewebe der menschlichen Bronchialschleimhaut für verschiedene Gewebezustände, beispielhaft für eine Anregungswellenlänge von 405 nm, über der Wellenlänge W in Nanometer,
- Fig. 2: die auf das erste Fluoreszenzmaximum im Grünen normierten Kurvenverläufe der spektralen Fluoreszenzintensitäten Iₛ aus Fig. 1,
- Fig. 3: schematisch den Aufbau der Diagnosevorrichtung,
- Fig. 4: die prinzipiellen spektralen Verläufe der Transmission T der den jeweiligen Sensoren einer konventionellen 3-Chip-Kamera vorgelagerten optischen Filter im Kopfprisma über der Wellenlänge W in Nanometer,
- Fig.5: der in einer konventionellen Bildaufnahmeeinheit mittels Filter erzeugte spektrale Verlauf der Transmission T am langwelligen Rand des sichtbaren Bereichs über der Wellenlänge W in Nanometer,
- Fig. 6: ein Ausführungsbeispiel für den spektralen Verlauf der Transmission T eines zusätzlich in den Bildpfad eingebrachten optischen Filters für eine zusätzliche Rotdämpfung über der Wellenlänge W in Nanometer,
- Fig. 7: der spektrale Verlauf der Transmission T des zu Fig. 6 optisch komplementären Filters, welches im Weißlichtmodus in den Strahlengang der Lichtquelle eingeschwenkt wird, über der Wellenlänge W in Nanometer,
- Fig. 8: der Verlauf der von der Bildaufnahmeeinheit detektierten, spektralen Fluoreszenzintensität 1, (willkürliche Einheiten) von Gewebe der menschlichen Bronchialschleimhaut für verschiedene Gewebezustände bei gleichzeitiger, zusätzlicher Beaufschlagung des Gewebes mit Rotlicht aus dem Anregungs- bzw. Beleuchtungspfad und optischer Rotdämpfung im Bildpfad über der Wellenlänge W in Nanometer,
- Fig. 9: der spektrale Verlauf der Transmission T des Fluoreszenzanregungsfilters mit zusätzlicher Rottransmission über dem sichtbaren Spektralbereich und
- Fig. 10: die Hellempfindlichkeitskurve (relative Augenempfindlichkeit) des menschlichen Auges über der Wellenlänge W in Nanometer.

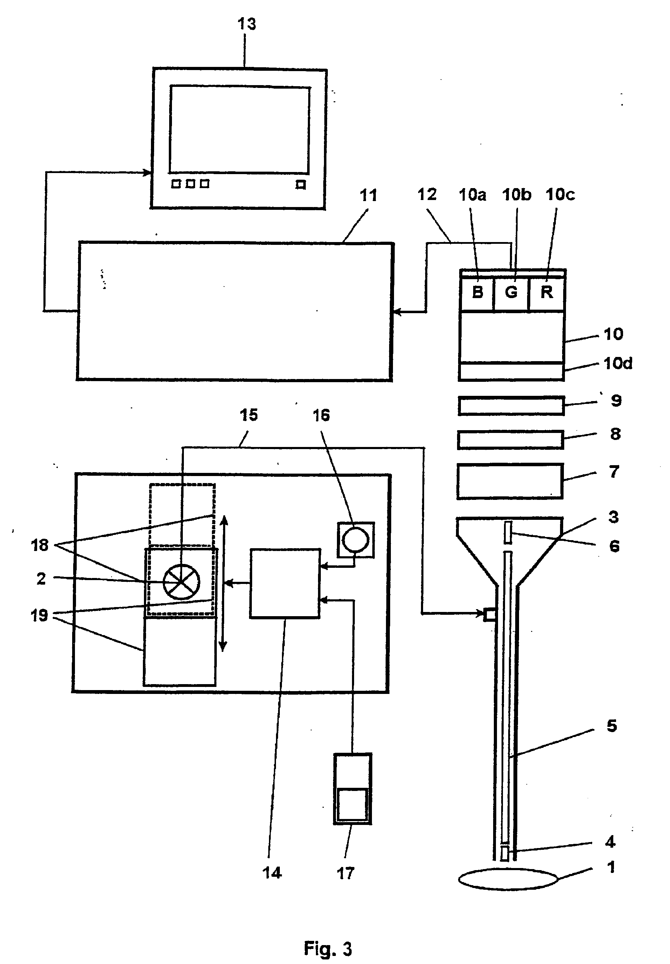

In Fig. 3 ist die Diagnosevorrichtung für die kombinierte bildgebende Weißlichtdiagnose und bildgebende Autofluoreszenzdiagnose zu sehen. Die Vorrichtung zur Untersuchung des Gewebes 1 weist eine Lichtquelle 2 auf, wobei eine inkohärente, breitbandig im sichtbaren Spektralbereich emittierende Lichtquelle besonders gut geeignet ist. Die idealerweise weißes Licht emittierende Lichtquelle 2 kann eine Kurzbogenlampe sein; hier ist sowohl eine Xenonlampe vorteilhaft als auch eine Gasentladungslampe mit Quecksilberanteilen oder eine Quecksilberhoch- oder gar Quecksilberhöchstdrucklampe. Letztere haben ein breites Strahlungsgrundkontinuum im Sichtbaren und die für das Quecksilber typischen Linien im Blauen und Violetten. Diese liegen zum Teil ideal im Absorptionsspektrum für die Fluoreszenzanregung der für die Autofluoreszenzdiagnose relevanten körpereigenen Fluorochrome. Alternativ kommt auch eine Halogenlampe in Betracht.

Alle genannten Lampen haben den Vorteil, dass sie einerseits einen Strahlungsanteil im Violetten / Blauen und einen, wenn auch bei manchen Lampen kleinen, Anteil im Roten besitzen und andererseits auch in der Lage sind, weißes Licht zu emittieren. Diese Lampen bieten damit ideale Voraussetzungen für ein System, welches sowohl die konventionelle Weißlichtdiagnose als auch die Autofluoreszenzdiagnose in ihrer hier beschriebenen Form ermöglichen soll.

Das Licht der Lichtquelle 2 gelangt über einen Lichtleiter 15 (Einzelfaser, Faserbündel oder Flüssiglichtleitkabel) zu Endoskop 3. Für die Lichteinkopplung in den Lichtleiter 15 kann die Lichtquelle 2 als Reflektorlampe ausgebildet sein. Denkbar ist jedoch auch eine Kondensoranordnung. Das Endoskop 3 leitet das Licht in bekannter Weise zum Gewebe 1. Mittels einer Bildübertragungseinheit wird das Bild des zum untersuchenden Gewebes 1 zur Bildaufnahmeeinheit geleitet, welche im vorliegenden Ausführungsbeispiel aus einem Kamerakopf 10 besteht. Mit 11 ist die Bildverarbeitungseinheit bezeichnet, also der zum Kamerakopf 10 gehörige Controller. Die Bildübertragungseinheit besteht im Ausführungsbeispiel aus einem (Endoskop-) Objektiv 4, einem Bildfaserbündel 5 oder alternativ aus einem Stab- oder GRIN- Linsensystem, einem Okular 6 und einem Kameraobjektiv 7.

Um im Modus der Autofluoreszenzdiagnose das vergleichsweise schwache Autofluoreszenzlicht gegenüber dem intensitätsstarken, am Gewebe remitterten Fluoreszenzanregunglicht überhaupt erst sichtbar werden zu lassen, ist irgendwo im Bildpfad, beispielsweise zwischen Kameraobjektiv 7 und Kamerakopf 10, ein Fluoreszenzanregungslicht-Blockfilter 8 eingebracht, welches dafür sorgt, dass das am Gewebe remittierte Fluoreszenzanregungslicht die Detektionseinheit der Kamera nicht erreichen kann.

Dieses Blockfilter kann im Bildpfad entweder fest oder ein- und ausschwenkbar angebracht sein. Dieses Blockfilter kann auch Teil des Endoskops 3 sein, beispielsweise dort im Okular untergebracht sein.

Bei der Kamera, bestehend aus Kamerakopf 10 und Controller 11, handelt es sich im vorliegenden Ausführungsbeispiel um eine konventionelle, beliebige endoskopische 3-Chip-Kamera, deren Kopf die drei Sensoren 10a, 10b und 10c aufweist, welche (in gängiger Weise) den drei spektralen Farbbereichen blau (B), grün (G) und rot (R) zugeordnet sind. Die Kamera kann damit ohne Einschränkungen, vor allen Dingen ohne Einbußen bei der Farbwiedergabe, für die konventionelle Weißlichtendoskopie benutzt werden. Sie kann als CCD-Kamera oder als CMOS-Kamera ausgeführt sein. Eine strahlaufteilende Einheit (nicht abgebildet) im Kamerakopf 10 zerlegt das dort ankommende Bild des zu untersuchenden Gewebes 1 in für 3-Chip-Kameras bekannter Weise in die drei Spektralbereiche "blau" (B), "grün" (G) und "rot" (R) und bildet dieses auf die Sensoren 10a, 10b und 10c ab.

Über die Signalleitungen 12 werden die Signale der drei Sensoren 10a, 10b und 10c zum Controller 11 geleitet. Von dort gelangen die Bildsignale zu Monitor 13, auf dem sich der Arzt das Bild des zu untersuchenden Gewebes 1 ansehen kann.

Die Vorrichtung weist eine Steuereinheit 14 auf, mit der die Diagnosemodi (Weißlichtdiagnose und Autofluoreszenzdiagnose) gesteuert bzw. geschaltet werden können. Diese Steuereinheit ist vorzugsweise in der Lichtquelle untergebracht. Soll beispielsweise vom Modus der Weißlichtdiagnose in den Modus der bildgebenden Autofluoreszenzdiagnose umgeschaltet werden, wird wie folgt vorgegangen:
Der Arzt betätigt einen Handschalter 16 oder einen Fußschalter 17. Das Schaltersignal wird zur Steuereinheit 14 geleitet. Diese sorgt dafür, dass ein Fluoreszenzanregungsfilter 18 in den Strahlengang der Lichtquelle 2 eingebracht wird. Auf diese Weise wird das zu untersuchende Gewebe 1 mit Fluoreszenzanregungslicht bestrahlt. Das Filter 18 ist so ausgeführt, dass neben dem Fluoreszenzanregungslicht - bei der angesprochenen Indikation im blauen / violetten Bereich - auch rotes Licht zum Gewebe gelangt. Das Gewebe wird also sowohl mit Fluoreszenzanregungslicht als auch zusätzlich mit Rotlicht bestrahlt.

Das erneute Betätigen der Schalter 16 bzw. 17 sorgt dafür, dass das Fluoreszenzanregungsfilter 18 aus dem Strahlengang der Lichtquelle 2 wieder ausgeschwenkt wird, so dass vom Modus der bildgebenden Autofluoreszenzdiagnose zur Weißlichtdiagnose zurückgeschaltet werden kann.

Mit der beschriebenen Diagnosevorrichtung wird also im Modus der bildgebenden Autofluoreszenzdiagnose das zu untersuchende Gewebe 1 - im Gegensatz zur "konventionellen" Autofluoreszenzdiagnose, bei welcher lediglich das Autofluoreszenzlicht selbst detektiert und bewertet wird -zusätzlich mit rotem Licht bestrahlt und das vom Gewebe remittierte Licht für die Gewebebewertung herangezogen unter Verwendung einer beliebigen, konventionellen endoskopischen 3-Chip-Kamera, die nicht in Kommunikation mit dem restlichen System und / oder einer zentralen Steuereinheit stehen muß und deshalb auch keine diagnosemodusbedingten Statusveränderungen beim Umschalten zwischen den beiden Arbeitsmoden erfahren muß. Im Ausführungsbeispiel transmittiert das Filter 18 in der Lichtquelle zusätzlich zum blauen / violetten Licht wie beschrieben rotes Licht. Alternativ zum dargestellten Aufbau kann jedoch auch eine separate Rotlichtquelle, beispielsweise ein Rotlicht-Laser, Verwendung finden.

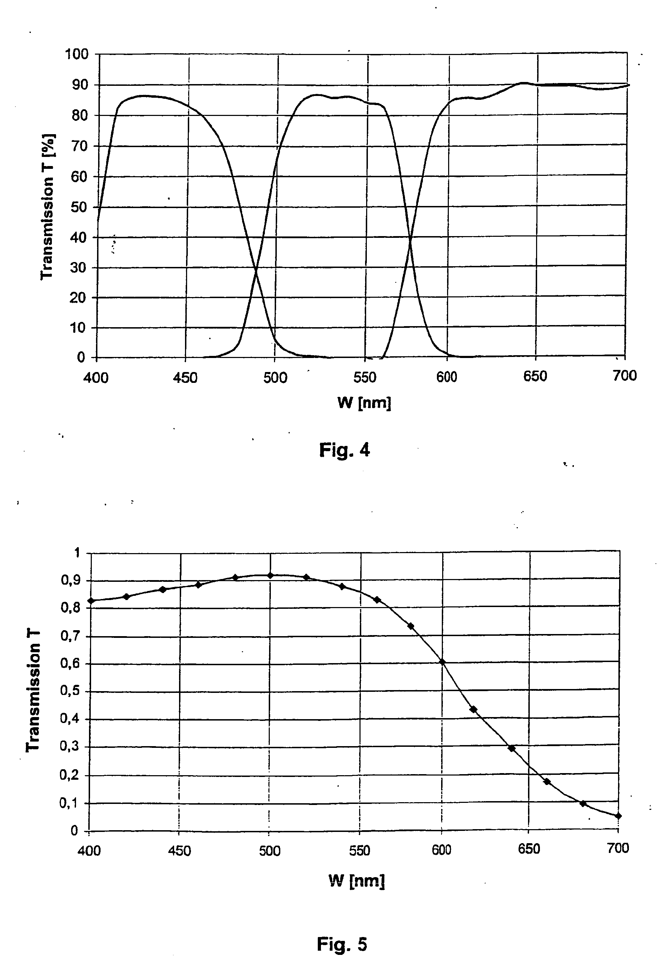

Die Vorgehensweise beim hier vorgestellten Verfahren und die damit erzielte Wirkung wird nachfolgend anhand der Figuren erläutert, wobei weiterhin die 3-Chip-Kamera des konkreten Ausführungsbeispiels zu Grunde gelegt wird:
Wie bereits an anderer Stelle erläutert, versuchen die meisten bekannten und verfügbaren Systeme für die bildgebende Autofluoreszenzdiagnose ein Maximum des vom Gewebe emittierten Fluoreszenzlichts zu detektieren. Dieser Optimierungsversuch gilt bei diesen Systemen auch für den roten Spektralbereich aus zweierlei Gründen: So wird erstens ein Maximum an Bildhelligkeit erzeugt und zweitens die Rotstichigkeit von (früh-) malignem Gewebe und damit die Farbdifferenzierung gegenüber gesundem, von grünem Fluorszenzlicht dominiertem Gewebe am besten dargestellt. Der Kopf einer konventionellen 3-Chip-Kamera enthält ein beschichtetes Prisma, welches eine Aufteilung der am Kopf eintreffenden Strahlung entsprechend den in der Fig. 4 abgebildeten Kurven vornimmt. Diese Kurven stellen das Transmissionsverhalten des Prismas für die drei unterschiedlichen, dem Prisma zugeordneten Sensoren ("Blausensor", "Grünsensor" und "Rotsensor") dar. Die Kurvenverläufe unterscheiden sich zwar von Hersteller zu Hersteller, beispielsweise hinsichtlich der Steilheit und exakten Lage der Filterkanten, geringfügig, sind aber im Hinblick auf ihr prinzipielles spektrales Verhalten identisch.

Insbesondere die nahezu gleichbleibend hohe Transmission im Langwelligen (Roten) beim "Rotsensor" der 3-Chip-Kamera kommt den genannten Bestrebungen, das differenzierte farbliche Verhalten von atypischem Gewebe (Rotstichigkeit) bestmöglich darzustellen und auch eine gute Bildhelligkeit zu erzeugen, entgegen. Ein Anbieter eines Autofluoreszenzdiagnosesystems, welcher eine farbbildtaugliche 3-Chip-Kamera einsetzt, nutzt auch tatsächlich aus besagten Gründen dieses spektrale Verhalten des Prismas aus, indem er dafür sorgt, dass die anderen optischen Systemkomponenten im Bildpfad und insbesondere diejenigen der Bildaufnahmeeinheit keine weiteren wesentlichen Änderungen des spektralen Transmissionsverhaltens erzeugen, also auch diese gute Rottransmission im Wesentlichen beibehalten.

Es wurde aber andererseits bereits ausführlich erläutert, dass nicht nur die Grünfluoreszenz gewebezustandsbezogenen Schwankungen unterworfen ist, sondern ungünstigerweise auch die Rotfluoreszenz (siehe dazu Fig. 1; die dort dargestellten Kurven wurden bei einer Fluoreszenzanregungswellenlänge von 405 nm ermittelt, wobei in einem dieser Wellenlänge benachbarten Anregungswellenlängenbereich die Ergebnisse ähnlich sind). Im Sinne einer verbesserten Sensitivität und Spezifität ist deshalb die Schaffung einer (konstanten) Rotreferenz anzustreben (weil dadurch wie bereits ausführlich erläutert eine verbesserte Farbdifferenzierung realisiert werden kann), welche außerdem quantitativ zwischen der Grünfluoreszenz von gesundem Gewebe und der Grünfluoreszenz von (früh-) malignem Gewebe liegt.

Deshalb wird bei der hier geschilderten Vorgehensweise der gewebezustandsabhängigen Rotfluoreszenzein entsprechend hoher Anteil zusätzlichen, gewebezustandsunabhängigen Rotlichts, welches von der Lichtquelle stammt und am Gewebe remittiert wird, überlagert und im Bildpfad, vor der Detektion durch den "Rotsensor", optisch gedämpft. Die Höhe der optischen Dämpfung wird bestimmt durch das Verhältnis von Fluoreszenzanregungslicht zu zusätzlich von der Lichtquelle bereitgestelltem Rotlicht. Die optische Dämpfung muß so eingestellt werden oder umgekehrt der Umfang des zusätzlichen Rotlichts an die optische Dämpfung so angepaßt werden, dass gesundes Gewebe grün und krankes Gewebe rot erscheint. Außerdem gilt: Je höher die optische Dämpfung im Bildpfad, um so stärker kann der Anteil des zusätzlichen Rotlichts von der Lichtquelle im Beleuchtungs- bzw. Anregungspfad gewählt werden. Je höher aber der Anteil des zusätzlichen, gewebezustandsunabhängigen Lichtquellen-Rotlichts gegenüber dem gewebezustandsabhängigem Rotfluoreszenzanteil ist, um so besser nähert man sich der gewebezustandsunabhängigen und damit optimierten Rotreferenz.

Ein erster und für ein nichtoptimiertes System schon ausreichender Schritt dahingehend kann die Verwendung einer konventionellen 3-Chip-Kamera mit einem gewöhnlichen Kopf sein. Bei einer 1-Chip-Kamera oder einem Videoendoskop sind die Verhältnisse vergleichbar und damit die Vorgehensweise entsprechend. Um eine Kompatibilität mit den Empfänger-Primärstrahlern genormter Systeme herzustellen [1], ist bei diesem konventionellen Kopf dem beschichteten Kopfprisma mit den in Fig. 4 dargestellten Transmissionskurven ein weiteres Filter vorgelagert, welches eine optische Dämpfung im Roten ausführt. Ein typisches Beispiel für das Transmissionsverhalten eines solchen Filters, welches in der Regel im Eingangsfensterbereich eines Kamerakopfes untergebracht ist oder dieses sogar bildet (10d in Fig. 3), ist in Fig. 5 dargestellt. Auch hier gibt es herstellergebunden geringfügige Unterschiede, während das prinzipielle Verhalten jedoch identisch und durch die genormten Empfänger-Primärstrahler vorgegeben ist. Die dämpfende Wirkung dieses Filters im Roten sorgt also bereits dafür, dass die vom Gewebe emittierte Rotfluoreszenz den "Rotsensor" nicht mehr in ihrem in Fig. 1 dargestellten Umfang erreichen kann, sondern bereits nur noch in abgeschwächter Form. Die Rotfluoreszenz spielt damit im Gesamtfluoreszenzbild bereits eine untergeordnete Rolle und (früh-) malignes Gewebe zeigt nicht mehr seine ursprünglich vorhandene, starke Rotstichigkeit, sondern erscheint nun mehr in stark abgedunkeltem, jedoch gründominiertem Licht.

Durch zusätzliche Überlagerung von Rotlicht, welches von der Lichtquelle zur Verfügung gestellt wird - im Ausführungsbeispiel durch entsprechende Spezifizierung des Fluoreszenzanregungsfilters 18 im Lichtprojektor- und am Gewebe remittiert wird, gelingt es nun, im Bild einen Rotanteil zu erzeugen, welcher schon bedeutend unabhängiger vom Gewebezustand ist, als der ursprünglich allein durch die Rotfluoreszenz erzeugte, und welcher damit der gewünschten optimalen Rotreferenz schon einen merklichen Schritt näher kommt. Die Menge dieses zusätzlichen Rotlichts wird einerseits bestimmt durch die Forderung, dass das gesunde Gewebe in einem kräftigen Grün und krankes Gewebe in einem deutlichen Rot erscheinen soll und andererseits durch das Ausmaß der dämpfenden Wirkung des Filters am Eingangsfenster des Kamerakopfes, welches selbstverständlich in gleicher Weise auch auf dieses zusätzliche und vom Gewebe remittierte Rotlicht wirkt. Durch die Bereitstellung von zusätzlichem Rotlicht ist es nun ausserdem möglich, krankes Gewebe in einem wesentlich helleren Rot darzustellen (ohne Einbußen im Signal-Rausch-Verhältnis), als wenn ausschließlich Fluorszenzlicht in die Gewebebewertung einbezogen wird, denn aus Fig. 1 wird auch ersichtlich, dass beim kranken Gewebe die Rotfluoreszenz kaum höher ist als die Grünfluoreszenz.

Eine weitere Reduktion der gewebezustandsbedingten Rotfluoreszenzschwankungen und damit einen weiteren Schritt in Richtung Etablierung einer echten optimierten Rotreferenz mit dem Ziel einer maximalen Sensitivität und Spezifität gelingt durch die Positionierung weiterer Mittel zur optischen Rotdämpfung im Bildpfad und dabei vorzugsweise nun in der Bildübertragungseinheit statt in der Bildaufnahmeeinheit, da es sich bei der Bildaufnahmeeinheit aus Systemkostengründen um eine beliebige konventionelle Komponente handeln soll und diese deshalb keinerlei Veränderungen erfahren soll.

Dies kann im einfachsten Fall durch das zusätzliche Einbringen eines weiteren Filterplättchens 9 in den Bildpfad, beispielsweise zwischen Blockfilter 8 und Kamerakopf 10 (siehe Fig. 3), geschehen. Bei dem Filterplättchen 9 kann es sich beispielsweise um ein beschichtetes Glasplättchen handeln. Die Beschichtung kann auch so spezifiziert sein, dass sie sowohl den Anforderungen an die Rotdämpfung und auch den Anforderungen an die Blockung des Fluoreszenzanregungslichts gerecht wird. Das zusätzliche Blockfilter 8 würde dann entfallen, es kann aber auch an einer anderen Stelle im Bildpfad, beispielsweise im Okular des Endoskops, untergebracht sein. Um aus Gründen einer bequemen Handhabung weitere separate Komponenten im System zu vermeiden, kann das Filterplättchen 9 auch Bestandteil einer ohnehin erforderlichen Systemkomponente sein, beispielsweise des Endoskops 3 oder des Kameraobjektivs 8. Allerdings muss berücksichtigt werden, dass dann diese Systemkomponenten zu speziellen Komponenten werden, d. h. es können dafür nicht mehr länger konventionelle Elemente verwendetwerden. Will man jedoch ein preiswertes System realisieren, welches insbesondere bei den teueren Bestandteilen (Kamera, Endoskop, ...) auf konventionelle und eventuell ohnehin bereits sich beim Anwender befindliche Bausteine zurückgreifen kann, dann sollte das Filter 9 in einem insofern günstigeren Element, beispielsweise im Kameraobjektiv 7, untergebracht sein. Neben dem Lichtprojektor wäre damit das Kameraobjektiv 7 das einzige spezielle Element des Systems, und die Anzahl der notwendigen Komponenten wäre außerdem auf das übliche beschränkt.

Auf andere Maßnahmen der optischen Rotdämpfung, wie z. B. die Beschichtung von sich im Bildpfad befindlichen optischen Bauteilen (gleichfalls mit reduzierter Rottransmission) oder die Verwendung von optischen Materialien, die im roten Spektralbereich eine reduzierte Transmission aufweisen, z. B. spezielle Gläser oder Bildleiterfasern, wurde an anderer Stelle bereits hingewiesen.

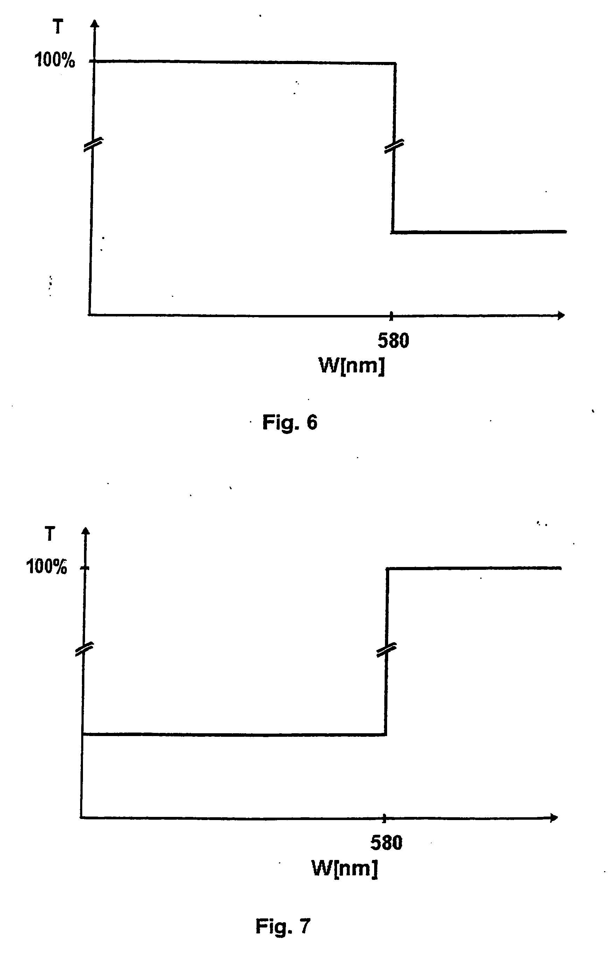

Realisiert man die optische Rotdämpfung über ein Filterplättchen, kann die Beschichtung im einfachsten Fall so spezifiziert sein, dass man im blauen und grünen Spektralbereich eine konstante Transmission von idealerweise nahezu hundert Prozent erreicht, während die Transmission im roten Spektralbereich lediglich bei einem Bruchteil von hundert Prozent liegt und in diesem Bereich gleichfalls konstant ist (siehe das Beispiel in Fig. 6). Andere Ausführungsformen des spektralen Transmissionsverhaltens des Filterplättchens sind gleichfalls denkbar.

Sollen die optischen Mittel für die Rotdämpfung, also beispielsweise das zusätzlich im Bildpfad untergebrachte, eventuell im Kameraobjektiv positionierte Filterplättchen, auch beim Wechsel in den Modus der Weisslichtdiagnose fest im Bildpfad positioniert bleiben, um so einen schnellen, einfachen und bequemen Wechsel zwischen den beiden Arbeitsmoden zu gewährleisten, dann erhält man dort, also im Modus der Weißlichtdiagnose, ohne weitere Maßnahmen zunächst ein cyanstichiges Farbbild, da die durch die Rotdämpfung verlorengegangenen Rotanteile fehlen. Ein automatischer Weißabgleich mit der Kamera schafft keine wirkliche Abhilfe: Die so herbeigeführte, die optische Rotdämpfung kompensierende elektronische Rotverstärkung durch die Bildverarbeitungseinheit kompensiert dann auch im Modus der Autofluoreszenzdiagnose die dort gewünschte Rotdämpfung, wenn in diesen Modus wieder zurückgeschaltet wird.

Echte Abhilfe kann dadurch geschaffen werden, dass in den Lichtprojektor ein weiteres optisches Filter, in Fig. 3 das Element 19, eingebracht wird. Dieses Filter 19 wird über die Steuereinheit 14 beim Umschalten in den Modus der Weißlichtdiagnose in den Strahlengang der Lichtquelle 2 eingeschwenkt. Wird in den Modus der Autofluoreszenzdiagnose umgeschaltet, wird über die Steuereinheit 14 das Filter 19 aus dem Strahlengang ausgeschwenkt und das Fluoreszenzanregungsfilter 18 mit der zusätzlichen Rottransmission in den Strahlengang eingeschwenkt.

Die Beschichtung dieses Filters 19 sollte idealerweise so gewählt werden, dass sich deren spektrales Transmissionsverhalten komplementär zum spektralen Transmissionsverlauf der Beschichtung von Filter 9 verhält, d. h., dass bei Überlagerung von Filter 9 und Filter 19 ein über das sichtbare Spektrum nahezu konstanter Transmissionsgrad resultieren sollte. Fig. 7 zeigt das spektrale Transmissionsverhalten von Filter 19, welches komplementär zum in Fig. 6 dargestellten, beispielhaften spektralen Transmissionsverlauf des Filters 9 ist. So gelingt es, im Modus der Weißlichtdiagnose ein optimales Farbbild ohne jegliche Farbstichigkeit zu generieren und im Modus der Autofluoreszenzdiagnose die gewünschte Rotdämpfung, welche optisch herbeigeführt wurde, beizubehalten.

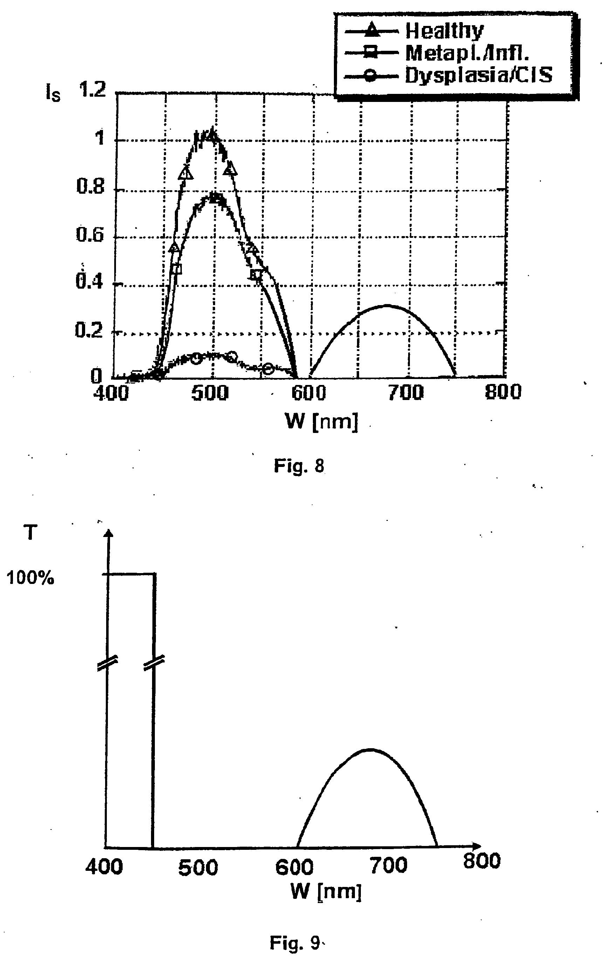

Die vorgeschlagenen Maßnahmen für die optische Rotdämpfung führen also dazu, dass der an der Sensoreinheit der Bildaufnahmeeinheit eintreffende Rotanteil der in Fig. 1 dargestellten Gewebsfluoreszenz stark reduziert wird und dementsprechend auch die vom Grad der Gewebeatypie abhängigen Schwankungen der Rotfluoreszenz im vom Monitor 13 dargestellten Bild keine Rolle mehr spielen (im Wesentlichen wird zunächst nur noch der grüne Fluoreszenzanteil dargestellt). Wird nun von der Lichtquelle im Modus der Autofluoreszenzdiagnose neben dem Fluoreszenzanregungslicht zusätzlich Rotlicht bereitgestellt in einem Umfang, dass sich nach der optischen Dämpfung dieses Rotlichts durch die im Bildpfad getroffenen Maßnahmen beispielhaft der in Fig. 8 dargestellte Kurvenverlauf für den Rotanteil ergibt (welcher nun nahezu unabhängig vom Grad der Gewebeatypie ist) mit den daraus resultierenden Rot-Grün-Intensitätsverhältnissen - diese Intensitätsverhältnisse entsprechen den Verhältnissen der Fläche unter der Kurve im roten Spektralbereich zu den Flächen unter den Kurven im grünen Spektralbereich - dann erreicht man, dass gesundes Gewebe grün ausschaut (klare Dominanz der Grünfluoreszenz gegenüber dem von der Lichtquelle zusätzlich abgegebenen und vom Gewebe remittierten Rotlicht) und im Vergleich dazu krankes Gewebe in einem hellen Rot erscheint (Dominanz des unverändert hohen Rotanteils gegenüber der nun deutlich reduzierten Grünfluoreszenz).

Fig. 9 zeigt beispielhaft das spektrale Transmissionsverhalten des im Modus der Autofluoreszenzdiagnose in den Strahlengang der Lichtquelle 2 eingeschwenkten Fluoreszenzanregungslichtfilters mit der zusätzlichen Rottransmission.

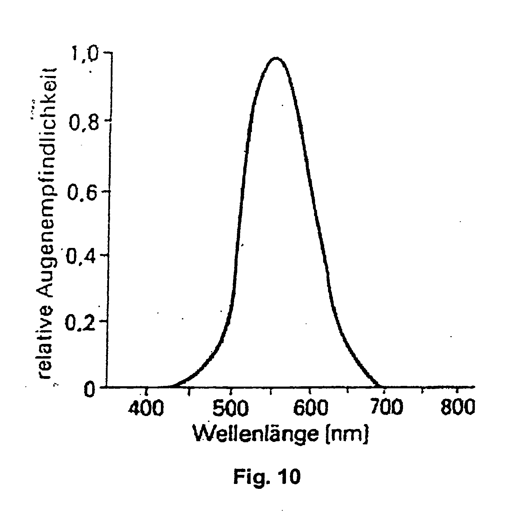

Dervorgeschlagene Lösungsweg liefert auch klare Vorteile beim endoskopischen Direkteinblick: Es ist in einfachster Weise möglich, auch im Modus der Autofluoreszenzdiagnose, auf Bildaufnahme- und Bildverarbeitungseinheiten, also beispielsweise auf eine Kamera, zu verzichten und dennoch die Vorzüge der gesteigerten Sensitivität und Spezifität zu genießen. Realisiert man zunächst den auch oben beschriebenen einfachsten Fall, dass die optische Rotdämpfung ausschließlich durch das sich im Engangsfensterbereich konventioneller Kameras befindliche Filter generiert wird ("eingeschränkte Rotdämpfung"), dann kann ohne zusätzliche Maßnahmen auch ein endoskopischer Direkteinblick vorgenommen werden, da die bei der Darstellung auf dem Monitor ausgeführte optische Rotdämpfung durch das besagte Filter beim Direkteinblick vom menschlichen Auge selbst vorgenommen wird. Dies wird deutlich, wenn man das langwellige spektrale Verhalten des Bildaufnahmeeinheit-Filters (Fig. 5) mit dem langwelligen spektralen Verhalten der Hellempfindlichkeitskurve des menschlichen Auges (Fig. 10) vergleicht: die Kurvenverläufe der Fig. 5 und Fig. 10 sind im roten Spektralbereich vergleichbar.

Werden weitere Maßnahmen für die optische Rotdämpfung ("erweiterte Rotdämpfung") ergriffen, um die gewebezustandsbedingten Rotfluoroszenz-Schwankungen nahezu vollständig zu eliminieren, dann muss nur dafür gesorgt werden, dass diese Maßnahmen auch beim endoskopischen Direkteinblick gewährleistet bleiben. Wird also beispielsweise ein zusätzliches Filterplättchen 9 mit reduzierter Rottransmission in den Bildpfad eingebracht, dann muss dies auch beim Direkteinblick im Strahlengang zwischen zu untersuchendem Gewebe und menschlichem Auge bleiben. War zum Beispiel dieses Filterplättchen bei der Bilddarstellung auf dem Monitor im Kameraobjektiv 7 untergebracht, dann muss es nun beim Direkteinblick, also ohne Kameraobjektiv 7, am Okulartrichter des Endoskops 3 angebracht werden. Ansonsten wäre das Bild zu rotstichig, da das Fluoreszenzanregungsfilter 18 und die dort spezifizierte Transmission des zusätzlichen Rotlichts u. a. auf das dämpfende Verhalten dieses Filterplättchens 9 abgestimmt wurde. Gleiches gilt für die alternativen Maßnahmen zur optischen Rotdämpfung.

Verwendet man statt der Kombination Endoskop - Kamera ein Videoendoskopsystem und beschränkt sich auf die "eingeschränkte Rotdämpfung" in der oben beschriebenen Form, dann kann ein beliebiges, konventionelles Videoendoskopsystem herangezogen werden. Will man die "erweiterte Rotdämpfung", dann kann zumindest beim Controller auf ein konventionelles Gerät zurückgegriffen werden. Beim Endoskop selbst muss dann vor der Sensoreinheit ein optisches Element mit einer reduzierten Rottransmission positioniert sein.

Der erfindungsgemäß vorgesehene Einbezug von vom Lichtprojektor im Modus der Autofluoreszenzdiagnose zusätzlich bereitgestelltem und vom Gewebe remittierten Rotlicht in die Gewebebewertung bei gleichzeitiger, darauf abgestimmter optischer Dämpfung des von der Bildübertragungseinheit übertragenen Rotlichts, bevor dieses die Sensoreinheit der Bildaufnahmeeinheit erreicht, in der hier beschriebenen Form hat also folgende Funktion und Vorteile:

Die Sensitivität für prä- bzw. frühmaligne Läsionen gegenüber benignem Gewebe wird gesteigert, indem der dem Detektor zugeführte Rotanteil nun nicht mehr oder kaum noch vom Grad der Gewebeatypie abhängig ist (im Gegensatzzu den Verhältnissen bei der reinen Fluoreszenzdetektion) und dementsprechend für einen verbesserten Farbkontrast zwischen benignem und frühmalignem Gewebe gesorgt ist, weil das frühmaligne Gewebe gegenüber dem benignen Gewebe deutlich röter erscheint.

Dem Rotlicht kommt mit dieser "Fixierung" auf einen bezüglich des Grades der Gewebeatypie nahezu unveränderlichen Wert gleichzeitig eine Referenzfunktion zu: Es ist nur noch die Änderung der Grünfluoreszenz für die Gewebebewertung von Bedeutung, da ja idealerweise das der Kamera und damit dem Betrachter zugeführte Rotlicht vom Gewebezustand nahezu unabhängig und diesbezüglich fast konstant ist. Bei entsprechend vorgenommener optischer Dämpfung ist das Grünfluoreszenzlicht beim gesunden Gewebe deutlich stärker als das detektierte Rotlicht und dieses erscheint deshalb in einem kräftigen Grün, beim kranken Gewebe hingegen ist das Grünfluoreszenzlicht deutlich schwächer als das detektierte Rotlicht und letzteres erscheint deshalb in einem kräftigen Rot.

Würde man allein diesen Aspekt berücksichtigen wollen, dass ohnehin nur noch die Änderung der Grünfluoreszenz betrachtet wird, wäre eine völlige Ausblendung des gesamten Rotlichts eine Alternative zur erläuterten Vorgehensweise. Der zusätzliche Aufwand der Festlegung von Filterspezifikationen für den Fluoreszenzanregungslichtfilter im Roten oder alternativ eine zusätzliche rote Lichtquelle unter Beibehaltung der üblichen Filterspezifikationen, d. h. die Anpassung des von der Lichtquelle bereitgestellten und am Gewebe remittierten Rotlichts an die optische Rotdämpfung und an den Umfang von verfügbarem Fluoreszenzanregungslicht, entfallen somit. Der Farbeindruck bzw. die Farbverschiebung zwischen grün und rot wiederum würde jedoch dann für die Gewebebewertung keine Rolle mehr spielen, nur noch der Intensitätsrückgang im Grünen wäre für die Gewebedifferenzierung relevant.

Wie jedoch bereits erwähnt wurde, kann auch gesundes Gewebe, dessen Oberfläche aufgrund morphologischer Unebenheiten wie eine "Lichtfalle" wirkt, einen ähnlichen Fluoreszenzrückgang verursachen wie prä- bzw. frühmalignes Gewebe, mit der Folge, dass hier kaum eine Differenzierung möglich ist, was dann zu einem Rückgang der Spezifität führt. Wird jedoch remittiertes Rotlicht in der beschriebenen Art in die Gewebebewertung mit einbezogen, findet die Gewebebewertung in erster Linie wieder über den dem Betrachter vermittelten Farbeindruck statt. Dieser ist unabhängig von der Oberflächenstruktur des Gewebes, da die Lichtfallenwirkung bei Rotlicht und Grünlicht gleichermaßen wirksam, also unabhängig von der Wellenlänge ist und unabhängig vom Abstand Endoskop - Gewebe. Wie gewünscht bewirkt also allein der Grad der Gewebeatypie eine Farbänderung. Insofern kommt dem zusätzlich detetkierten, remittierten Rotlicht im Vergleich zum völligen Verzicht jeglicher Rotlichtdetektion eine wichtige Referenzfunktion zu.

Schließlich liefert das zusätzliche, von der Lichtquelle zur Verfügung gestellte und vom Gewebe remittierte Rotlicht gegenüber der Maßnahme des völligen Ausblendens von Rotlicht aber auch gegenüber der alleinigen Detektion von Fluoreszenzlicht über den gesamten Spektralbereich (Grün- und Rotfluoreszenz) einen zusätzlichen Helligkeitsgewinn. Gegenüber dem letzteren Fall besteht die Möglichkeit, durch eine vergleichsweise starke optische Rotdämpfung und eine daran angepaßte, entsprechend hohe zusätzliche Rotlichtmenge von der Lichtquelle das Signal-Rausch-Verhältnis in diesem Spektralbereich zu verbessern. Vor dem Hintergrund der ohnehin intensitätschwachen Fluoreszenzbilder und der in der Regel gegenüber der Empfindlichkeit im Grünen geringeren Empfindlichkeit konventioneller medizinischer Bildaufnahmeeinheiten im Roten -bedingt durch das vor der Sensoreinheit angebrachte optische Filter mit reduzierter Rottransmission, welches die Kompatibilität dieser konventionellen Farbkameras mit den genormten Empfänger-Primärstrahlern herstellt, siehe oben und [1]) - ist dieser Aspekt von größter Bedeutung. Es können also ohne andere die Bildqualität beeinträchtigende Maßnahmen, wie elektronische Verstärkung oder Anheben der Bildintegrationszeit, eine Helligkeitsanhebung im Autofluoreszenzmodus und eine Verbesserung des Signal-Rausch-Verhältnisses erzielt werden.

Ein weiterer Vorteil der beschriebenen Anordnung, insbesondere gegenüber dem in der US 5 590 660 beschriebenen Diagnosesystem, besteht darin, dass insbesondere bei der "erweiterten Rotdämpfung" der gesamte Spektralbereich, bei welchem die spektralen Intesitätsunterschiede zwischen benignem und frühmalignem Gewebe gering sind, durch die optische Rotdämpfung "ausgeblendet" wird und durch eine Rotreferenz ersetzt wird, während nur der relativ schmale spektrale Bereich, bei welchem diese Intensitätsunterschiede groß sind (Wellenlängen kleiner als 580 nm) auch dargestellt werden. Dies resultiert in einer vergleichsweise starken, gewebezustandsbedingten Schwankung der Grünfluoreszenz gegenüber der Rotreferenz und deshalb in einer farblichen Kontrastanhebung gegenüber der in der US 5 590 660 beschriebenen Vorgehensweise, bei welcher ein wesentlich breiterer spektraler Bereich bis zu Wellenlängen von 650 nm, in welchem dann integral die gewebezustandsbedingten Schwankungen der Fluoreszenz weniger ausgeprägt sind, dem Grünkanal des Monitors zugeführt werden (Falschfarbenbild). Die Farbdifferenzierung wird dadurch "verwaschener" und die Möglichkeiten der Gewebedifferenzierung ungünstiger.

Die zur Realisierung der erläuterten Vorgehensweise beschriebene Vorrichtung erlaubt ferner in vorteilhafter Weise die Verwendung einer einzigen konventionellen Bildaufnahmeeinheit für beide Arbeitsmodi (Weißlicht und Autofluoreszenz) und damit zusammenhängend einen einfachen Wechsel zwischen den beiden Arbeitsmodi der Weißlichtdiagnose und der bildgebenden Autofluoreszenzdiagnose zu realisieren. Eine spezielle Sensor- und Filterkonzeption der Bildaufnahmeeinheit und damit eine Abweichung von der für die Weißlichtdiagnose optimalen optischen Konfiguration sind nicht erforderlich, d. h., es bestehen hinsichtlich der Bild- und Farbqualität auch im Weißlichtmodus optimale Voraussetzungen für die Erzeugung guter Bilder. Da die Diagnosevorrichtung nur eine Bildaufnahme- und Bildverarbeitungseinheit für beide Diagnosemodi benötigt, ist die Voraussetzung für ein kleines, leichtes, handliches und preiswertes System geschaffen, das trotzdem eine sehr gute Sensitivität und eine hohe Spezifität aufweist.

Da die Maßnahmen zur Rotdämpfung im Bereich der Optik liegen, also nicht Bestandteil der Elektronik sind, kann auch eine beliebige, konventionelle endoskopische Bildverarbeitungseinheit Verwendung finden. In dieser müssen nun nicht mehr länger diagnosemodusbedingte Umschaltvorgänge (z. B. Wechsel der Rotkanalsignalverstärkung) vorgenommen werden. Eine diesbezügliche Ansteuerung der Bildverarbeitungseinheit ist also nicht erforderlich.

Nur dadurch ist es möglich, jede beliebige, konventionelle endoskopische Bildaufnahme- und Bildverarbeitungseinheit in dieses System miteinzubeziehen. Es ist beispielsweise nicht mehr länger eine spezielle Kamera mit ansteuerbarem Controller erforderlich. Dies senkt die Systemkosten erheblich, da der Anwender auf evtl. vorhandene Geräte zurückgreifen kann. Es gibt kein anderes System, welches die an anderer Stelle ausführlich beschriebenen Vorteile (verbesserte Sensitivität und Spezifität, einfachstes Handling mit leichter und schneller Umschaltmöglichkeit zwischen den Diagnosemethoden und dergleichen) aufweist und gleichzeitig beispielsweise jede beliebige, konventionelle endoskopische Kamera verwenden kann. Bei den anderen bekannten Systemen werden immer Kameras mitangeboten, welche Bestandteil des Systems sind und an die mit der entsprechenden Vorgehensweise zusammenhängenden Erfordernisse angepaßt wurden. Dies ist auch in den selteneren Ausnahmen der Fall, wenn es sich um farbbildtaugliche Kameras handelt.

Bei dervorgestellten Vorgehensweise der optischen Rotdämpfung besteht ausserdem nicht die Gefahr der Sensorsättigung, was sich durch ein zu helles Monitorbild andeuten würde. Das Licht wird nämlich nun bereits gedämpft, bevor es die Sensoreinheit erreicht.

Da das neben dem Anregungslicht im Autofluoreszenzmodus zusätzliche, für die Farbkontrastanhebung verwendete Licht im roten Spektralbereich liegt, ist die Eindringtiefe vergleichsweise hoch, der gerichtet reflektierte Anteil vergleichsweise niedrig und damit der erzielte Farbeindruck des Gewebes nahezu unabhängig vom Winkel Endoskop - Gewebenormale im Gegensatz zu Systemen, welche blaues Fluoreszenzanregungslicht detektieren und für die Gewebebewertung miteinbeziehen. Der Effekt kann noch dadurch gesteigert werden, indem das spektrale Band des von der Lichtquelle 2 zusätzlich emittierten Rotlichts schmal gewählt wird und an den langwelligen Rand des Sichtbaren gelegt wird.

### Literatur

[1] Mäusl, Rudolf: "Fernsehtechnik:Übertragungsverfahren für Bild, Ton und Daten"; 2., überarbeitete und erweiterte Auflage; Hüthig-Verlag, 1995

## Patentansprüche

1. Vorrichtung zur bildgebenden Diagnose von Gewebe (1) unter wahlweiser Anwendung von zwei Diagnosemethoden, nämlich einem Arbeitsmodus zur bildgebenden Weißlichtdiagnose und einem Arbeitsmodus zur bildgebenden Autofluoreszenzdiagnose, mit einer Lichtquelle (2), deren Licht über ein Endoskop (3) zum Gewebe (1) geleitet wird, und mit einer Bildübertragungseinheit und einer Bildaufnahmeeinheit (10), welche mit einer Bildverarbeitungseinheit (11) in Verbindung steht, über welche ein Monitor (13) mit einem Bildsignal versorgt wird, wobei die Lichtquelle (2) im Arbeitsmodus der bildgebenden Autofluoreszenzdiagnose neben Fluoreszenzanregungslicht im blauen/violettem Spektralbereich zusätzliche Rotlicht emittiert, **dadurch gekennzeichnet, dass** das Rotlicht so intensiv ist, dass das vom Gewebe remittierte Rotlichts die Gewebefluoreszenz dominiert, und dass das System im Bildpfad optische Mittel (9) aufweist, welche das Rofficht so dämpfen, dass dieses quantitativ zwischen der relativ starken Grünfluoreszenz von gesundem Gewebe und der stark reduzierten Grünfluoreszenz von prä- bzw. frühmalignem Gewebe liegt.

2. Vorrichtung nach Anspruch 1, **dadurch gekennzeichnet, dass** die Bildaufnahmeeinheit aus einem Kamerakopf (10) und die Bildverarbeitungseinheit aus einem Kameracontroller (11) besteht.

3. Vorrichtung nach einem der Ansprüche 1 und 2, **dadurch gekennzeichnet, dass** die optischen Mittel zur Rotdämpfung Teil des Kamerakopfes (10) und/oder Teil der Bildübertragungseinheit sind.

4. Vorrichtung nach Anspruch 1, **dadurch gekennzeichnet, dass** die Bildaufnahmeeinheit aus dem Sensorsystem eines Videoendoskops (3) und die Bildverarbeitungseinheit (11) aus dem zugehörigen Controller besteht.

5. Vorrichtung nach Anspruch 4, **dadurch gekennzeichnet, dass** die optischen Mittel zur Rotdämpfung Teil des Sensorsystems des Videoendoskopes (3) und/oder Teil der Bildübertragungseinheit des Videoendoskopes (3) sind.

6. Vorrichtung nach einem der Ansprüche 1 bis 3, **dadurch gekennzeichnet, dass** die optischen Mittel zur Rotdämpfung durch ein Bildfaserbündel mit einer reduzierten Transmission im roten Spektralbereich gebildet sind.

7. Vorrichtung nach einem der Ansprüche 1 bis 5, **dadurch gekennzeichnet, dass** die optischen Mittel zur Rotdämpfung aus einer Beschichtung einer oder mehrerer optischer Komponenten der Bildübertragungseinheit und/oder der Bildaufnahmeeinheit (10) bestehen, wobei die Beschichtung eine reduzierte Transmission im roten Spektralbereich aufweist.

8. Vorrichtung noch einem der Ansprüche 1 bis 5, **dadurch gekennzeichnet. dass** die optischen Mittel zur Rotdämpfung ein Filferplättchen (9) in der Bildübertragungseinheit und/oder der Bildaufnahmeeinheit (10) mit einer reduzierten Transmission im roten Spektrcilbereich sind.

9. Vorrichtung nach einem der Ansprüche 1 und 8, **dadurch gekennzeichnet, dass** die Dämpfung durch die optischen Mittel so stark ist, dass im Modus der bildgebenden Autofluoreszenzdiagnose benignes Gewebe grün und prä- bzw. frühmalignes Gewebe rot erscheint.

10. Vorrichtung nach einem der Ansprüche 1 bis 9, **dadurch gekennzeichnet. dass** die Lichtquelle (2) aus einer inkohärenten, spektral breitbandigen Lichtquelle besteht, die ihr Licht zumindest zeitweise über einen optischen Filter (18) abgibt, der eine Lichttransmission im blauen/violetten Spektralbereich und im roten Spektralbereich erlaubt.

11. Vorrichtung nach einem der Ansprüche 1 bis 10, **dadurch gekennzeichnet, dass** die Lichtquelle (2) aus einer inkohärenten, spektral breitbandigen Lichtquelle und einer zusätzlichen Rotlichtquelle besteht.

12. Vorrichtung noch Anspruch 11. **dadurch gekennzeichnet, dass** die Rotlichtquelle aus einem Laser, welcher im roten Spektralbereich emittiert, besteht.

13. Vorrichtung nach Anspruch 11. **dadurch gekennzeichnet, dass** die Rotlichtquelle aus einer einzelnen LED oder einem Array von LED's, welche im roten Spektralbereich emittieren, besteht.

14. Vorrichtung nach einem der Ansprüche 1 bis 10, **dadurch gekennzeichnet, dass** die Lichtquelle (2) aus einer einzelnen LED oder einem Array von LED's, welche im blauen/violetten Spektralbereich emittieren, und einer Rotlichtquelle besteht.

15. Vorrichtung nach einem der Ansprüche 1 bis 10, **dadurch gekennzeichnet, dass** die Lichtquelle (2) aus einem Laser, welcher im blauen/violetten Spektralbereich emittiert, und einer Rotlichtquelle besteht.

16. Vorrichtung nach einem der Ansprüche 14 oder 15, **dadurch gekennzeichnet, dass** die Lichtquelle (2) weiterhin eine einzelne LED oder einen Array von im grünen Spektralbereich emittierenden LED's aufweist.

17. Vorrichtung nach einem der Ansprüche 2 und 4, **dadurch gekennzeichnet, dass** es sich bei den Controllern der Bildaufnahmeeinheit (10) um konventionelle Controller handelt.

## Claims

1. A device for the imaging diagnosis of tissue (1) under the selective application of two diagnosis methods, specifically an operating mode for the imaging, white-light diagnosis, and an operating mode for imaging, auto-fluorescence diagnosis, with a light source (2) whose light is led to the tissue (1) via an endoscope (3), and with a picture transmission unit and a picture recording unit (10) which is in connection with a picture-processing unit (11), via which a monitor (13) is supplied with a picture signal, wherein the light source (2) in the operating mode of the imaging, auto-flourescence diagnosis, apart from fluorescence excitation light in the blue/violet spectral range additionally emits red light, **characterised in that** the red light is so intensive, that the red light remitted from the tissue dominates the tissue fluorescence, and that the system in the picture path comprises optical means (9) which dampen the red light, such that this quantitatively lies between the relatively strong green fluorescence of healthy tissue and the greatly reduced green fluorescence of premalignant or early malignant tissue.

2. A device according to claim 1, **characterised in that** the picture recording unit consists of a camera head (10), and the picture processing unit of a camera controller (11).

3. A device according to one of the claims 1 and 2, **characterised in that** the optical means for red damping are part of the camera head (10) and/or part of the picture transmission unit.

4. A device according to claim 1, **characterised in that** the picture recording unit consists of the sensor system of a video endoscope (3), and the picture processing unit (11) of the associated controller.

5. A device according to claim 4, **characterised in that** the optical means for red damping are part of the sensor system of the video endoscope (3) and/or part of the picture transmission unit of the video endoscope (3).

6. A device according to on e of the claim 1 to 3, **characterised in that** the optical means for red damping are formed by a picture fibre bundle with a reduced transmission in the red spectral range.

7. A device according to one of the claims 1 to 5, **characterised in that** the optical means for red damping consist of a layering of one or more optical components of the picture transmission unit and/or of the picture recording unit (10), wherein the layering has a reduced transmission in the red spectral range.

8. A device according to one of the claim 1 to 5, **characterised in that** the optical means for red damping are a filter platelet (9) in the picture transmission unit and/or in the picture recording unit (10), with a reduced transmission in the red spectral range.

9. A device according to one of the claims 1 and 8, **characterised in that** the damping by way of the optical means is so great, that in the mode of the imaging, auto-flourescence diagnosis, benign tissue appears green, and premalignant or early malignant tissue appears red.

10. A device according to one of the claim 1 to 9, **characterised in that** the light source (2) consists of an incoherent, spectral, broadband light source which emits its light at least partly via an optical filter (18), which permits a light transmission in the blue/violet spectral range and in the red spectral range.

11. A device according to one of the claims 1 to 10, **characterised in that** the light source (2) consists of an incoherent, spectrally broadband light source and of an additional red light source.

12. A device according to claim 11, **characterised in that** the red light source consist of a laser which emits in the red spectral range.

13. A device according to claim 11, **characterised in that** the red light source consists of an individual LED or of an array of LED's which emit in the red spectral range.

14. A device according to one of the claims 1 to 10, **characterised in that** the light source (2) consist of an individual LED or an array of LED's which emit in the blue/violet spectral range, and of a red light source.

15. A device according to one of the claim 1 to 10, **characterised in that** the light source (2) consists of a laser which emits in the blue/violet spectral range, and of a red light source.

16. A device according to one of the claims 14 or 15, **characterised in that** the light source (2) further comprises an individual LED or an array of LED's emitting in the green spectral range.

17. A device according to one of the claim 2 and 4, **characterised in that** with the controllers of the picture recording unit (10), it is the case of conventional controllers.

## Revendications

1. Dispositif d'imagerie pour le diagnostic de tissu (1 ) par application au choix de deux méthodes de diagnostic, à savoir un mode opératoire pour le diagnostic d'imagerie en lumière blanche et un mode opératoire pour le diagnostic d'imagerie en autofluorescence, comportant une source lumineuse (2), dont la lumière est transmise au tissu (1) via un endoscope (3), et une unité de transmission de l'image et une unité de prise de vues (10), reliée à une unité de traitement de l'image (11), par l'intermédiaire de laquelle un moniteur (13) reçoit un signal d'image, la source lumineuse (2), dans le mode de diagnostic d'imagerie en autofluorescence, émettant, outre une lumière d'excitation de la fluorescence dans la plage spectrale bleu/violet, également une lumière rouge, **caractérisé en ce que** la lumière rouge est d'intensité telle que la lumière rouge retransmise par le tissu domine la fluorescence tissulaire, et que le système présente, sur le trajet lumineux, des moyens optiques (9) qui atténuent la lumière rouge de telle sorte que celle-ci se situe quantitativement entre la fluorescence verte relativement forte du tissu sain et la fluorescence verte fortement réduite du tissu pré-malin.

2. Dispositif selon la revendication 1, **caractérisé en ce que** l'unité de prise de vues consiste en une tête de caméra (10) et l'unité de traitement de l'image en un contrôleur de caméra (11).

3. Dispositif selon l'une des revendications 1 et 2, **caractérisé en ce que** les moyens optiques d'atténuation de la lumière rouge font partie de la tête de caméra (10) et/ou font partie de l'unité de transmission de l'image.

4. Dispositif selon la revendication 1, **caractérisé en ce que** l'unité de prise de vues est constituée du système de capteur d'un vidéo-endoscope (3) et l'unité de traitement de l'image (11) du contrôleur associé.

5. Dispositif selon la revendication 4, **caractérisé en ce que** les moyens optiques d'atténuation de la lumière rouge font partie du système de capteur du vidéo-endoscope (3) et/ou font partie de l'unité de transmission de l'image du vidéo-endoscope (3).

6. Dispositif selon l'une des revendications 1 à 3, **caractérisé en ce que** les moyens optiques d'atténuation de la lumière rouge sont formés par un faisceau de fibres optiques à transmission réduite dans la plage spectrale rouge.

7. Dispositif selon l'une des revendications 1 à 5, **caractérisé en ce que** les moyens optiques d'atténuation de la lumière rouge consistent en un revêtement d'un ou plusieurs composants optiques de l'unité de transmission de l'image et/ou de l'unité de prise de vues (10), le revêtement présentant une transmission réduite dans la plage spectrale rouge.

8. Dispositif selon l'une des revendications 1 à 5, **caractérisé en ce que** les moyens optiques d'atténuation de la lumière rouge sont une plaquette filtrante (9) dans l'unité de transmission de l'image et/ou dans l'unité de prise de vues (10) à transmission réduite dans la plage spectrale rouge.

9. Dispositif selon l'une des revendications 1 à 8, **caractérisé en ce que** l'atténuation par les moyens optiques est de force telle que, dans le mode de diagnostic d'imagerie en autofluorescence, le tissu bénin apparaît vert et le tissu pré-malin apparaît rouge.

10. Dispositif selon l'une des revendications 1 à 9, **caractérisé en ce que** la source lumineuse (2) consiste en une source lumineuse incohérente à larges bandes spectrales qui émet sa lumière au moins par instants via un filtre optique (18) qui autorise une transmission de la lumière dans la plage spectrale bleu/violet et dans la plage spectrale rouge.

11. Dispositif selon l'une des revendications 1 à 10, **caractérisé en ce que** la source lumineuse (2) consiste en une source lumineuse incohérente à larges bandes spectrales et en une source de lumière rouge additionnelle.

12. Dispositif selon la revendication 11, **caractérisé en ce que** la source de lumière rouge consiste en un laser qui émet dans la plage spectrale rouge.

13. Dispositif selon la revendication 11, **caractérisé en ce que** la source de lumière rouge consiste en une LED individuelle ou un groupe de LEDs, qui émettent dans la plage spectrale rouge.

14. Dispositif selon l'une des revendications 1 à 10, **caractérisé en ce que** la source lumineuse (2) consiste en une LED individuelle ou un groupe de LEDs, qui émettent dans la plage spectrale bleu/violet, et en une source de lumière rouge.

15. Dispositif selon l'une des revendications 1 à 10, **caractérisé en ce que** la source lumineuse (2) consiste en un laser qui émet dans la plage spectrale bleu/violet, et en une source de lumière rouge.

16. Dispositif selon l'une des revendications 14 ou 15, **caractérisé en ce que** la source lumineuse (2) présente, en outre, une LED individuelle ou un groupe de LEDs émettant dans la plage spectrale verte.

17. Dispositif selon l'une des revendications 2 et 4, **caractérisé en ce que** les contrôleurs de l'unité de prise de vues (10) sont des contrôleurs traditionnels.
